# EUROPEAN PATENT APPLICATION

(11) **EP 1 561 817 A2**
(43) Date of publication of application: **10.08.2005**
(21) Application number: 05004454.4
(22) Date of filing: 27.07.2001
(51) Int. Cl.: C12N 15/12, C12N 15/86, C12N 7/01, C12N 5/10, C07K 14/705, A61K 38/17, A61K 48/00

(54) **Modified CEA and uses thereof**

(30) Priority: 31.07.2000 US 222043 P
(62) Divisional of application: 01956237.0
(71) Applicant: Aventis Pasteur Limited, Toronto, Ontario M2R 3T4 (CA); Therion Biologics, Cambridge, MA 02142 (US); National Cancer Institute, Rockville, MD 20852 (US)
(72) Inventor: Berinstein, Neil, Toronto, Ontario M5P 1V1 (CA); Tartaglia, James, Aurora, Ontario L4G 6R6 (CA); Tine, John A., Scotia, NY 12302 (US); Panicali, Dennis L., Cambridge, MA 02142 (US); Gritz, Linda, Cambridge, MA 02142 (US); Schlom, Jeffrey, Potomac, MD 20854 (US)
(74) Representative: Nachshen, Neil Jacob

(57) **Abstract**

The invention discloses immunogenic CEA agonist polypeptides/proteins comprising a modified epitope containing the amino acid sequence YLSGADLNL, nucleic acids coding therefor, vectors and/or cells comprising said nucleic acids, and mixtures and/or compositions thereof. Methods for eliciting or inducing CEA-specific immune responses utilizing the aforementioned agents are also disclosed.

SEQUENCE LISTING

<110> AVENTIS PASTEUR LIMITED
      THERION BIOLOGICS
      NATIONAL CANCER INSTITUTE
      Berinstein, Neil
      Tartaglia, James
      Tine, John, A.
      Panicali, Dennis L.
      Gritz, Linda
      Schlom, Jeffrey

<120> MODIFIED CEA AND USES THEREOF

<130> 1038-1144 LAB

<150> US 60/222,042
<151> 31/07/2000

<160> 8

<170> PatentIn Ver. 2.0

<210> 1
<211> 701
<212> PRT
<213> CEA Modified Polypeptide

<400> 1

Met Glu Ser Pro Ser Ala Pro Pro His Arg Trp Cys Ile Pro Trp Gln Arg Leu Leu Leu
                5                   10                  15                  20

Thr Ala Ser Leu Leu Thr Phe Trp Asn Pro Pro Thr Thr Ala Lys Leu Thr Ile Glu Ser
                25                  30                  35                  40

Thr Pro Phe Asn Val Ala Glu Gly Lys Glu Val Leu Leu Leu Val His Asn Leu Pro Gln
                45                  50                  55                  60

His Leu Phe Gly Tyr Ser Trp Tyr Lys Gly Glu Arg Val Asp Gly Asn Arg Gln Ile Ile
                65                  70                  75                  80

Gly Tyr Val Ile Gly Thr Gln Gln Ala Thr Pro Gly Pro Ala Tyr Ser Gly Arg Glu Ile
                85                  90                  95                  100

Ile Tyr Pro Asn Ala Ser Leu Leu Ile Gln Asn Ile Ile Gln Asn Asp Thr Gly Phe Tyr
                105                 110                 115                 120

Thr Leu His Val Ile Lys Ser Asp Leu Val Asn Glu Glu Ala Thr Gly Gln Phe Arg Val
                125                 130                 135                 140

Tyr Pro Glu Leu Pro Lys Pro Ser Ile Ser Ser Asn Asn Ser Lys Pro Val Glu Asp Lys
                145                 150                 155                 160

Asp Ala Val Ala Phe Thr Cys Glu Pro Glu Thr Gln Asp Ala Thr Tyr Leu Trp Trp Val
                165                 170                 175                 180

Asn Asn Gln Ser Leu Pro Val Ser Pro Arg Leu Gln Leu Ser Asn Gly Asn Arg Thr Leu
                185                 190                 195                 200

Thr Leu Phe Asn Val Thr Arg Asn Asp Thr Ala Ser Tyr Lys Cys Glu Thr Gln Asn Pro
                205                 210                 215                 220

Tyr Ser Ala Arg Arg Ser Asp Ser Val Ile Leu Asn Val Leu Tyr Gly Pro Asp Ala Pro
                225                 230                 235                 240

Thr Ile Ser Pro Leu Asn Thr Ser Tyr Arg Ser Gly Glu Asn Leu Asn Leu Ser Cys His
                245                 250                 255                 260

Ala Ala Ser Asn Pro Pro Ala Gln Tyr Ser Trp Phe Val Asn Gly Thr Phe Gln Gln Ser
                265                 270                 275                 280

Thr Gln Glu Leu Phe Ile Pro Asn Ile Thr Val Asn Asn Ser Gly Ser Tyr Thr Cys Gln
                285                 290                 295                 300

Ala His Asn Ser Asp Thr Gly Leu Asn Arg Thr Thr Val Thr Thr Ile Thr Val Tyr Glu
                305                 310                 315                 320

Pro Pro Lys Pro Phe Ile Thr Ser Asn Asn Ser Asn Pro Val Glu Asp Glu Asp Ala Val
                325                 330                 335                 340

Ala Leu Thr Cys Glu Pro Glu Ile Gln Asn Thr Thr Tyr Leu Trp Trp Val Asn Asn Gln
                345                 350                 355                 360

Ser Leu Pro Val Ser Pro Pro Leu Gln Leu Ser Asn Asp Asn Arg Thr Leu Thr Leu Leu
                365                 370                 375                 380

Ser Val Thr Arg Asn Asp Val Gly Pro Tyr Glu Cys Gly Ile Gln Asn Glu Leu Ser Val
                385                 390                 395                 400

Asp His Ser Asp Pro Val Ile Leu Asn Val Leu Tyr Gly Pro Asp Asp Pro Thr Ile Ser
                405                 410                 415                 420

Pro Ser Tyr Thr Tyr Tyr Arg Pro Gly Val Asn Leu Ser Leu Ser Cys His Ala Ala Ser
                425                 430                 435                 440

Asn Pro Pro Ala Gln Tyr Ser Trp Leu Ile Asp Gly Asn Ile Gln Gln His Thr Gln Glu
                445                 450                 455                 460

Leu Phe Ile Ser Asn Ile Thr Glu Lys Asn Ser Gly Leu Tyr Thr Cys Gln Ala Asn Asn
                465                 470                 475                 480

Ser Ala Ser Gly His Ser Arg Thr Thr Val Lys Thr Ile Thr Val Ser Ala Glu Leu Pro
                485                 490                 495                 500

Lys Pro Ser Ile Ser Ser Asn Asn Ser Lys Pro Val Glu Asp Lys Asp Ala Val Ala Phe
                505                 510                 515                 520

Thr Cys Glu Pro Glu Ala Gln Asn Thr Thr Tyr Leu Trp Trp Val Asn Gly Gln Ser Leu
                525                 530                 535                 540

Pro Val Ser Pro Arg Leu Gln Leu Ser Asn Gly Asn Arg Thr Leu Thr Leu Phe Asn Val
                545                 550                 555                 560

Thr Arg Asn Asp Ala Arg Ala Tyr Val Cys Gly Ile Gln Asn Ser Val Ser Ala Asn Arg
                565                 570                 575                 580

Ser Asp Pro Val Thr Leu Asp Val Leu Tyr Gly Pro Asp Thr Pro Ile Ile Ser Pro Pro
                585                 590                 595                 600

Asp Ser Ser Tyr Leu Ser Gly Ala Asp Leu Asn Leu Ser Cys His Ser Ala Ser Asn Pro
                605                 610                 615                 620

Ser Pro Gln Tyr Ser Trp Arg Ile Asn Gly Ile Pro Gln Gln His Thr Gln Val Leu Phe
                625                 630                 635                 640

Ile Ala Lys Ile Thr Pro Asn Asn Asn Gly Thr Tyr Ala Cys Phe Val Ser Asn Leu Ala
                645                 650                 655                 660

Thr Gly Arg Asn Asn Ser Ile Val Lys Ser Ile Thr Val Ser Ala Ser Gly Thr Ser Pro
                665                 670                 675                 680

Gly Leu Ser Ala Gly Ala Thr Val Gly Ile Met Ile Gly Val Leu Val Gly Val Ala Leu
                685                 690                 695                 700

Ile

<210> 2
<211> 2106
<212> DNA
<213> CEA nucleic acid

<400> 2

atggagtctc cctcggccc tccccacaga tggtgcatcc cctggcagag gctcctgctc 60

acagcctcac ttctaacctt ctggaacccg cccaccactg ccaagctcac tattgaatcc 120

acgccgttca atgtcgcaga ggggaaggag gtgcttctac ttgtccacaa tctgccccag 180

cttctttttg gctacagctg gtacaaaggt gaaagagtgg atggcaaccg tcaaattata 240

ggatatgtaa taggaactca acaagctacc ccagggcccg catacagtgg tcgagagata 300

atatacccca atgcatccct gctgatccag aacatcatcc agaatgacac aggattctac 360

accctacacg tcataaagtc agatcttgtg aatgaagaag caactggcca gttccgggta 420

tacccggagc tgcccaagcc ctccatctcc agcaacaact ccaaacccgt ggaggacaag 480

gatgctgtgg ccttcacctg tgaacctgag actcaggacg caacctacct gtggtgggta 540

aacaatcaga gcctcccggt cagtcccagg ctgcagctgt ccaatggcaa caggaccctc 600
actctattca atgtcacaag aaatgacaca gcaagctaca aatgtgaaac ccagaaccca 660
gtgagtgcca ggcgcagtga ttcagtcatc ctgaatgtcc tctatggccc ggatgccccc 720
accatttccc ctctaaacac atcttacaga tcaggggaaa atctgaacct ctcctgccac 780
gcagcctcta acccacctgc acagtactct tggtttgtca atgggacttt ccagcaatcc 840
acccaagagc tctttatccc caacatcact gtgaataata gtggatccta tacgtgccaa 900
gcccataact cagacactgg cctcaatagg accacagtca cgacgatcac agtctatgag 960
ccacccaaac ccttcatcac cagcaacaac tccaaccccg tggaggatga ggatgctgta 1020
gccttaacct gtgaacctga gattcagaac acaacctccc tgtggtgggt aaataatcag 1080
agcctcccgg tcagtcccag gctgcagctg tccaatgaca acaggaccct cactctactc 1140
agtgtcacaa ggaatgatgt aggaccctat gagtgtggaa tccagaacga attaagtgtt 1200
gaccacagcg acccagtcat cctgaatgtc ctctatggcc cagacgaccc caccatttcc 1260
ccctcataca acctattacc tccaggggtg aacctcagcc tctcctgcca tgcagcctct 1320
aacccacctg cacagtattc ttggctgatt gatgggaaca tccagcaaca cacacaagag 1380
ctctttatct ccaacttcac tgagaagaac agcggactct attactgcca ggccaataac 1440
tcagccagtg gccacagcag gactacagtc aagacaatca cagtctctgc ggagctgccc 1500
aagccctcca tctccagcaa caactccaaa cccgtggagg acaaggatgc tgtggccttc 1560
acctgtgaac ctgaggctca gaacacaacc tacctgtggt gggtaaatgg tcagagcctc 1620
ccagtcagtc ccaggctgca gctgtccaat ggcaacagga ccctcactct attcaatgtc 1680
acaagaattg acgcaagagc ctatgtatgt ggaatccaga actcagtgag tgcaaaccgc 1740
agtgacccag tcaccctgga tgtcctctat gggccggaca cccccatcat ttccccccca 1800
gactcgtctt acctttcggg agcggacctc aacctctcct gccactcggc ctctaaccca 1860
tccccgcagt attcttggcg tatcaatggg ataccgcagc aacacacaca agttctcttt 1920
atcgccaaaa tcacgccaat taataacggg acctatgcct gttttgtctc taacttggct 1980
actggccgca ataattccat agtcaagagc atcacagtct ctgcatctgg aacttctcct 2040
ggtctctcag ctggggccac tgtcggcatc atgattggag tgctggttgg ggttgctctg 2100
atatag
2106

<210> 3
<211> 60
<212> DNA

<213> primer

<400> 3

ttgtccgagc tctcgcgata tccgttaagt ttgtatcgta atggagtctc cctcggcccc 60

<210> 4
<211> 31
<212> DNA
<213> primer

<400> 4

ccggaattct cacaagatct gactttatga c 31

<210> 5
<211> 23
<212> DNA
<213> primer

<400> 5

gatcgtcgac gagctcgaat tcg 23

<210> 6
<211> 23
<212> DNA
<213> primer

<400> 6

gatccgaatt cgagctcgtc gac 23

<210> 7
<211> 105
<212> DNA
<213> primer

<400> 7

ttgtccgagc tcgaattctt tattgggaag aatatgataa tattttggga tttcaaaatt 60

gaaaatatat aattacaata taaaatgggc cacacacgga ggcag 105

<210> 8
<211> 30
<212> DNA
<213> primer

<400> 8

acggcagtcg acttatacag ggcgtacact 30

SEQUENCE LISTING

<110> AVENTIS PASTEUR LIMITED
      THERION BIOLOGICS
      NATIONAL CANCER INSTITUTE
      Berinstein, Neil
      Tartaglia, James
      Tine, John A.
      Panicali, Dennis L.
      Gritz, Linda
      Schlom, Jeffrey

<120>  Modified CEA and Uses Thereof

<130>  P016281EPA

<140>  EP05004454.4
<141>  2001-07-27

<150>  60/222,042
<151>  2000-07-31

<160>  10

<170>  PatentIn version 3.2

<210>  1
<211>  701
<212>  PRT
<213>  CEA Modified Polypeptide

<400>  1

Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Glu
305                 310                 315                 320

Pro Pro Lys Pro Phe Ile Thr Ser Asn Asn Ser Asn Pro Val Glu Asp
                325                 330                 335

Glu Asp Ala Val Ala Leu Thr Cys Glu Pro Glu Ile Gln Asn Thr Thr
            340                 345                 350

Tyr Leu Trp Trp Val Asn Asn Gln Ser Leu Pro Val Ser Pro Arg Leu

        355                 360                 365

Gln Leu Ser Asn Asp Asn Arg Thr Leu Thr Leu Leu Ser Val Thr Arg
    370                 375                 380

Asn Asp Val Gly Pro Tyr Glu Cys Gly Ile Gln Asn Glu Leu Ser Val
385                 390                 395                 400

Asp His Ser Asp Pro Val Ile Leu Asn Val Leu Tyr Gly Pro Asp Asp
                405                 410                 415

Pro Thr Ile Ser Pro Ser Tyr Thr Tyr Tyr Arg Pro Gly Val Asn Leu
            420                 425                 430

Ser Leu Ser Cys His Ala Ala Ser Asn Pro Pro Ala Gln Tyr Ser Trp
        435                 440                 445

Leu Ile Asp Gly Asn Ile Gln Gln His Thr Gln Glu Leu Phe Ile Ser
    450                 455                 460

Asn Ile Thr Glu Lys Asn Ser Gly Leu Tyr Thr Cys Gln Ala Asn Asn
465                 470                 475                 480

Ser Ala Ser Gly His Ser Arg Thr Thr Val Lys Thr Ile Thr Val Ser
                485                 490                 495

Ala Glu Leu Pro Lys Pro Ser Ile Ser Ser Asn Asn Ser Lys Pro Val
            500                 505                 510

Glu Asp Lys Asp Ala Val Ala Phe Thr Cys Glu Pro Glu Ala Gln Asn
        515                 520                 525

Thr Thr Tyr Leu Trp Trp Val Asn Gly Gln Ser Leu Pro Val Ser Pro
    530                 535                 540

Arg Leu Gln Leu Ser Asn Gly Asn Arg Thr Leu Thr Leu Phe Asn Val
545                 550                 555                 560

Thr Arg Asn Asp Ala Arg Ala Tyr Val Cys Gly Ile Gln Asn Ser Val
                565                 570                 575

Ser Ala Asn Arg Ser Asp Pro Val Thr Leu Asp Val Leu Tyr Gly Pro
            580                 585                 590

Asp Thr Pro Ile Ile Ser Pro Pro Asp Ser Ser Tyr Leu Ser Gly Ala
        595                 600                 605

Asp Leu Asn Leu Ser Cys His Ser Ala Ser Asn Pro Ser Pro Gln Tyr
    610                 615                 620

Ser Trp Arg Ile Asn Gly Ile Pro Gln Gln His Thr Gln Val Leu Phe
625                 630                 635                 640

Ile Ala Lys Ile Thr Pro Asn Asn Asn Gly Thr Tyr Ala Cys Phe Val
                645                 650                 655

Ser Asn Leu Ala Thr Gly Arg Asn Asn Ser Ile Val Lys Ser Ile Thr
            660                 665                 670

Val Ser Ala Ser Gly Thr Ser Pro Gly Leu Ser Ala Gly Ala Thr Val
        675                 680                 685

Gly Ile Met Ile Gly Val Leu Val Gly Val Ala Leu Ile
    690                 695                 700

<210>  2
<211>  2106
<212>  DNA
<213>  CEA nucleic acid

<400>  2
atggagtctc cctcggcccc tccccacaga tggtgcatcc cctggcagag gctcctgctc      60

acagcctcac ttctaacctt ctggaacccg cccaccactg ccaagctcac tattgaatcc     120

acgccgttca atgtcgcaga ggggaaggag gtgcttctac ttgtccacaa tctgccccag     180

catctttttg gctacagctg gtacaaaggt gaaagagtgg atggcaaccg tcaaattata     240

ggatatgtaa taggaactca acaagctacc ccagggcccg catacagtgg tcgagagata     300

atatacccca atgcatccct gctgatccag aacatcatcc agaatgacac aggattctac     360

accctacacg tcataaagtc agatcttgtg aatgaagaag caactggcca gttccgggta     420

tacccggagc tgcccaagcc ctccatctcc agcaacaact ccaaacccgt ggaggacaag     480

gatgctgtgg ccttcacctg tgaacctgag actcaggacg caacctacct gtggtgggta     540

aacaatcaga gcctcccggt cagtcccagg ctgcagctgt ccaatggcaa caggaccctc     600

actctattca atgtcacaag aaatgacaca gcaagctaca aatgtgaaac ccagaaccca     660

gtgagtgcca ggcgcagtga ttcagtcatc ctgaatgtcc tctatggccc ggatgccccc     720

accatttccc ctctaaacac atcttacaga tcaggggaaa atctgaacct ctcctgccac     780

gcagcctcta acccacctgc acagtactct tggtttgtca atgggacttt ccagcaatcc     840

acccaagagc tctttatccc caacatcact gtgaataata gtggatccta tacgtgccaa     900

gcccataact cagacactgg cctcaatagg accacagtca cgacgatcac agtctatgag 960

ccacccaaac ccttcatcac cagcaacaac tccaaccccg tggaggatga ggatgctgta 1020

gccttaacct gtgaacctga gattcagaac acaacctacc tgtggtgggt aaataatcag 1080

agcctcccgg tcagtcccag gctgcagctg tccaatgaca acaggaccct cactctactc 1140

agtgtcacaa ggaatgatgt aggaccctat gagtgtggaa tccagaacga attaagtgtt 1200

gaccacagcg acccagtcat cctgaatgtc ctctatggcc cagacgaccc caccatttcc 1260

ccctcataca cctattaccg tccaggggtg aacctcagcc tctcctgcca tgcagcctct 1320

aacccacctg cacagtattc ttggctgatt gatgggaaca tccagcaaca cacacaagag 1380

ctctttatct ccaacatcac tgagaagaac agcggactct atacctgcca ggccaataac 1440

tcagccagtg gccacagcag gactacagtc aagacaatca cagtctctgc ggagctgccc 1500

aagccctcca tctccagcaa caactccaaa cccgtggagg acaaggatgc tgtggccttc 1560

acctgtgaac ctgaggctca gaacacaacc tacctgtggt gggtaaatgg tcagagcctc 1620

ccagtcagtc ccaggctgca gctgtccaat ggcaacagga ccctcactct attcaatgtc 1680

acaagaaatg acgcaagagc ctatgtatgt ggaatccaga actcagtgag tgcaaaccgc 1740

agtgacccag tcaccctgga tgtcctctat gggccggaca cccccatcat ttccccccca 1800

gactcgtctt acctttcggg agcggacctc aacctctcct gccactcggc ctctaaccca 1860

tccccgcagt attcttggcg tatcaatggg ataccgcagc aacacacaca agttctcttt 1920

atcgccaaaa tcacgccaaa taataacggg acctatgcct gttttgtctc taacttggct 1980

actggccgca ataattccat agtcaagagc atcacagtct ctgcatctgg aacttctcct 2040

ggtctctcag ctggggccac tgtcggcatc atgattggag tgctggttgg ggttgctctg 2100

atatag 2106

<210> 3
<211> 60
<212> DNA
<213> primer

<400> 3
ttgtccgagc tctcgcgata tccgttaagt ttgtatcgta atggagtctc cctcggcccc 60

<210> 4
<211> 31
<212> DNA
<213> primer

<400> 4

ccggaattct cacaagatct gactttatga c                                    31

<210>  5
<211>  23
<212>  DNA
<213>  primer

<400>  5
gatcgtcgac gagctcgaat tcg                                              23

<210>  6
<211>  23
<212>  DNA
<213>  primer

<400>  6
gatccgaatt cgacctcgtc gac                                              23

<210>  7
<211>  105
<212>  DNA
<213>  primer

<400>  7
ttgtccgagc tcgaattctt tattgggaag aatatgataa tattttggga tttcaaaatt      60

gaaaatatat aattacaata taaaatgggc cacacacgga ggcag                     105

<210>  8
<211>  30
<212>  DNA
<213>  primer

<400>  8
acggcagtcg acttatacag ggcgtacact                                       30

## Description

### FIELD OF THE INVENTION

The present invention relates to immunology, in particular to novel biologically active modified CEA agonist polypeptides/proteins containing a modified epitope therein, nucleic acids coding therefor, vectors and/or cells comprising said nucleic acid, mixtures and/or compositions of the aforementioned, and their use as immunogenic agents and/or in treatments of cancer.

### BACKGROUND OF THE INVENTION

Throughout this application, various references are cited in parentheses to describe more fully the state of the art to which this invention pertains. The disclosure of these references are hereby incorporated by reference into the present disclosure.

The prospects of cancer immunotherapy rely upon the identification of tumor associated antigens which can be recognized by the immune system. Specifically, target antigens eliciting T cell-mediated responses are of critical interest. This comes from evidence that cytotoxic T lymphocytes (CILs) can induce tumor regression both in animal models (Kast W. et al (1989) Cell 59:6035; Greendberg P. *(1991)Adv. Immunol.* 49:281) and in humans (Boon T. et al. (1994) *Annu. Rev. Immunol* 12:337).

Human carcinoembryonic antigen (CEA) is a 180 kD glycoprotein expressed on the majority of colon, rectal, stomach and pancreatic tumors (Muaro et al. (1985) *Cancer* Res. 45:5769), some 50% of breast carcinomas (Steward et al. (1974) Cancer 33:1246) and 70% of lung carcinomas (Vincent, R.G. and Chu, T.M. (1978) *J. Thor. Cardiovas.* Surg. 66:320). CEA is also expressed in fetal gut tissue and to a lesser extent on normal colon epithelium. The immunogenicity of CEA has been ambiguous, with several studies reporting the presence of anti-CEA antibodies in patients (Gold et al. (1973) *Nature New Biology* 239:60; Pompecki, R. (1980) *Eur. J. Cancer* 16:973; Ura et al. (1985) *Cancer Lett.25:283;* Fuchs et al. (1988) *Cancer Immunol. Immunother.* 26:180) while other studies have not (LoGerfo et al. (1972) *Int. J Cancer* 9:344; MacSween, J.M. (1975) *Int J. Cancer* 15:246; Chester K.A. and Begent, H.J. (1984) *Clin. Exp. Immunol* 58:685). CEA was first described as a cancer specific fetal antigen in adenocarcinoma of the human digestive tract in 1965 (Gold, P. and Freeman, 5.0. (1965) *Exp. Med.* 121:439). Since that time, CEA has been characterized as a cell surface antigen produced in excess in nearly all solid tumors of the human gastrointestinal tract. The gene for the human CEA protein has been cloned (Oikawa et al (1987) *Biochim. Biophys. Res.* 142:511-518; European Application No. EP 0346710).

Notwithstanding the aforementioned ambiguous demonstration of CEA's immunogenicity, a phase I clinical trail using a vaccinia-CEA vaccine ("rV-CEA") did demonstrate that CEA-specific cytolytic T-lymphocyte (CTL) response could be elicited in humans (Tsang, K.Y. et al. (1995) *J. Natl. Cancer lnstit* 87:982-990; Tsang K.Y. et al. (1997) *Clin. Cancer Res.* 3:2439-2449). As a consequence of the studies, a CEA immunodominant CTL epitope was identified. This 9-mer (i.e. YLSGANLNL) was shown to bind to a HLA-A2 class I molecule and has been designated carcinoembryonic antigen peptide-1 ("CAP-1"). Several subsequent studies have also demonstrated the ability of the CAP-I epitope/peptide *per se* to elicit CEA-specific human CTL responses (Alters, S.E. et al. (1998) *J. Immunother*. 21:17-26; Tsang, K.Y. et al. (1997) *Supra;* Zaremba, S. et al. (1997) *Cancer Res.* 57:4570-4577). Moreover, stable CTL lines derived by culture of peripheral blood mononuclear cells (PBMCs) from rV-CEA vaccinated patients with CAP1 and interleukin (IL)-2 have recently been described (Tsang, K.Y. (1997) *Supra)*.

It is an accepted principle that when an immunogenic peptide is modified in a conserved manner (i.e. a hydrophobic amino acid is substituted with a hydrophobic amino acid) the modified peptide is likely to have similar immunogenic activity based upon the maintenance of the molecule's shape, charge and hydrophobic character. More specifically, a study by Madden (Madden et al. (1993) *Cell* 75:693) has identified specific amino acid preferences in peptides for MHC-complexing, a precursor step to T cell recognition. Madden as well as other investigators (Rammensee et al., (1995) *Immunogenetics* 41:178) have suggested that specific amino acid positions in peptides are available for T cell recognition.

Skipper et al. ((1996) *J. Exp, Med.* 183: 527) described the identification and characterization of a naturally-occurring peptide epitope of tyrosinase wherein the peptide sequence differed from that which is predicted from the DNA. This modified peptide was recognized by tyrosinase-specific human cytotoxic T-lymphocytes ("CTL") more effectively than the direct translation product, and was the only one of the two peptides to be presented by HLA-A2.1 molecules on the cell surface. The modification was a substitution of an asparagine with an aspartic acid. The authors proposed that the asparagine was N-glycosylated in the endoplasmic reticulum during protein synthesis and subsequently deamidated post-translationally.

With respect to the CEA epitope CAP1, the primary and secondary anchors positions for HLA binding at positions 2, 9, and 1 (of the epitope) are already occupied by preferred amino acids. As such, Schlom and colleagues attempted to increase CAP1 immunogenicity via the generation of epitope analogs containing single amino acid substitutions to residues predicted to interact with the T cell receptor ('TCR") of CAP1-specific CTL. One such analog epitope (YLSGADLNL; designated CAP-1-6D) was identified which demonstrated an increased immunogenicity to that of the natural epitope, but not a concomitant increase in MHC binding *per se* (i.e. it behaved as an agonist; Zaremba, S. et al (1997) *Supra)*.

The present invention discloses novel modified CEA agonist polypeptides/proteins containing a modified epitope therein, nucleic acids coding therefor, vectors comprising said nucleic acids, mixtures and/or compositions of the aforementioned agents, and their advantageous use in generating CEA-specific immune responses and/or in the treatment of cancers.

### SUMMARY OF THE INVENTION

The present invention encompasses novel modified CEA agonist polypeptides/proteins comprising a modified epitope containing the sequence YLSGADLNL, nucleic acids coding therefore, vectors (such as recombinant virus and/or bacteria) and/or cells (such as antigen-presenting cells) comprising said nucleic acids, and mixtures and/or compositions of the aforementioned. All of these aforementioned agents, mixtures and compositions are characterized by their ability to induce or elicit an immune response against a CEA protein or fragment thereof, a CEA agonist polypeptide containing said modified epitope, a normal or modified CEA epitope, or cells binding or expressing the aforementioned CEA protein/fragment, CEA agonist polypeptide, or normal/modified CEA epitope.

Accordingly, in one embodiment of the invention a CEA agonist polypeptide/protein is provided comprising a modified epitope of CEA, wherein said modified epitope contains the sequence YLSGADLNL.

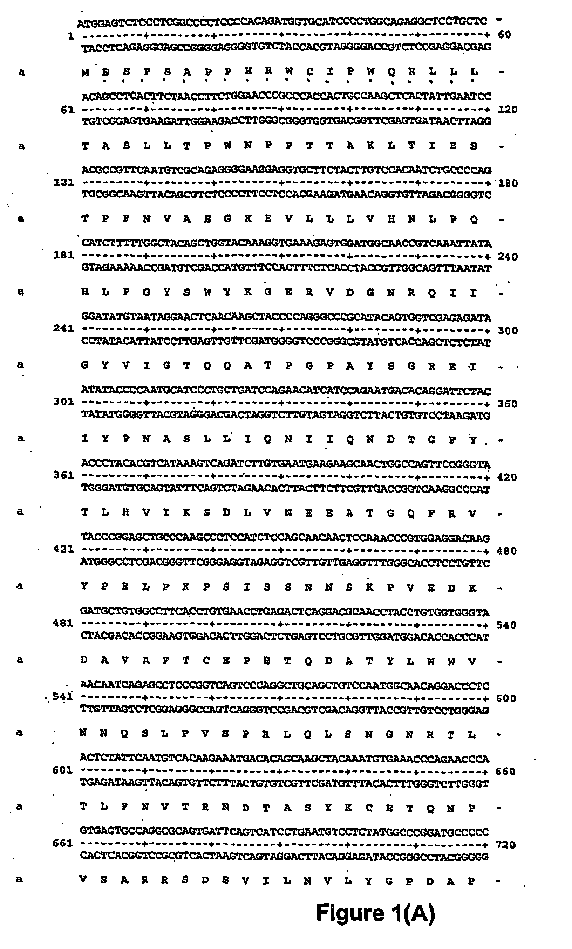

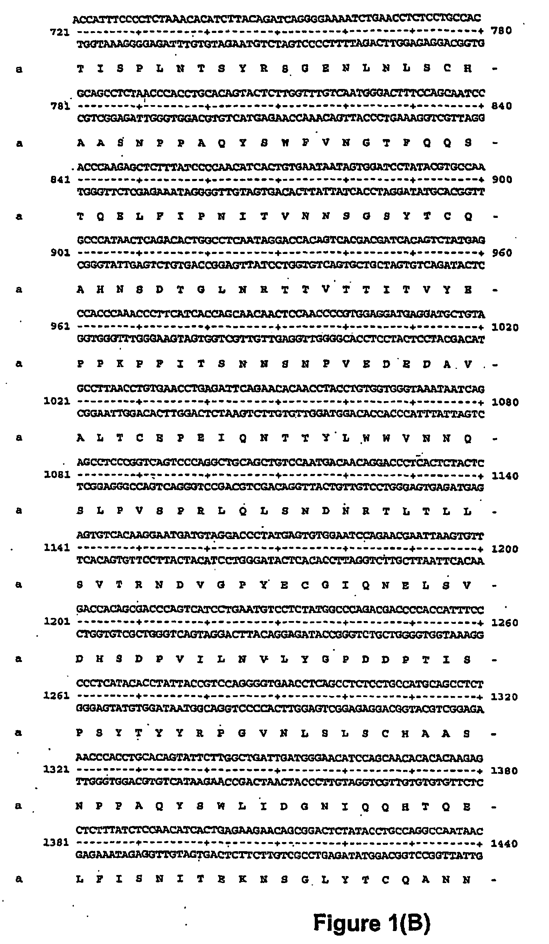

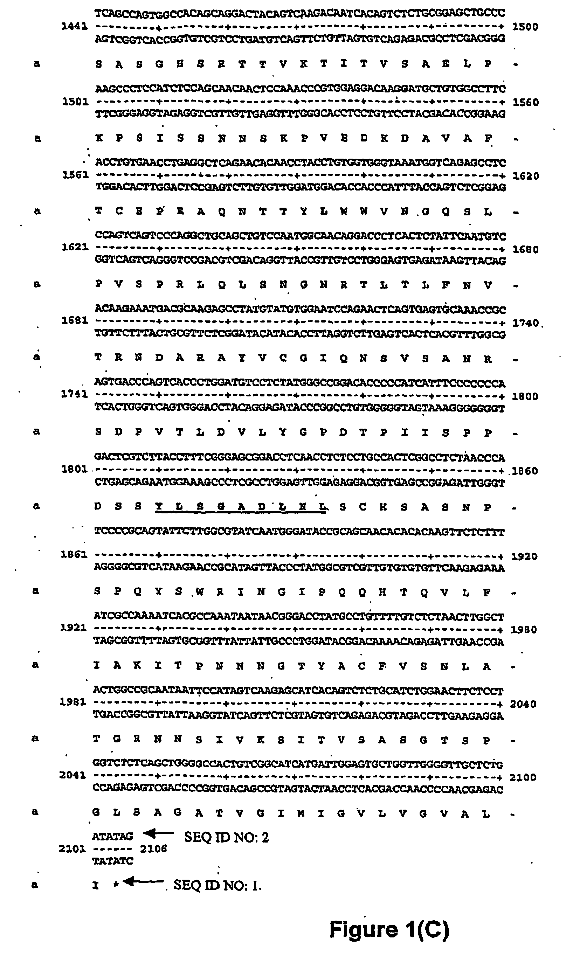

In a further embodiment of the invention, the CEA agonist polypeptide/protein has the amino acid sequence of SEQ ID NO: 1 (Figure 1).

As previously noted, embodiments of the invention encompass nucleic acids coding for the aforementioned CEA agonist polypeptides/proteins. Accordingly, embodiments of the invention consist/comprise the nucleic acid sequence of SEQ ID NO: 2 (Figure 1). In further embodiments of the invention, the nucleic acid is a DNA selected from the group consisting of viral nucleic acid, plasmid, bacterial DNA, naked/free DNA, and RNA. In yet further embodiments, the viral nucleic acid is selected from the group consisting of adenovirus, alphavirus and poxvirus. In still yet further embodiments, the poxvirus is selected from the group consisting of avipox, suipox and orthopox. In still yet further embodiments, the poxviral nucleic•acid is selected from the group consisting of TROVAC, NYVAC, ALVAC, MVA, Adeno-Associated Virus (AAV), Wyeth; and PoxvacTC.

Additional embodiments of the invention are contemplated encompassing nucleic acids comprising a sequence encoding for the CEA agonist polypeptide/protein in addition to a second sequence encoding at least one member selected from the group comprising cytokines, lymphokines, and co-stimulatory molecules.

Embodiments of the invention further contemplate vectors comprising the nucleic acid(s) of the invention. In particular embodiments, these vectors may be either recombinant viruses or bacteria. In further embodiments, the recombinant viruses are selected from the group consisting of adenovirus, alphavirus and poxvirus. In yet further embodiments, the poxvirus is selected for the group consisting of avipox, orthopox and suipox; particular embodiments encompass ALVAC, NYVAC, TROVAC, MVA, Wyeth and Poxvac-TC.

The invention further provides for cells comprising the aforementioned nucleic acid(s) of the invention, wherein said cells express the CEA agonist polypeptide/protein of the invention. In further embodiments, the cells expressing the CEA agonist polypeptide/protein also express a MHC HLA class 1 molecule. In yet further embodiments, the cells expressing the polypeptide are antigen-presenting cells.

Embodiments of the invention further encompass mixtures and/or compositions of the aforementioned CEA agonist polypeptides/proteins, nucleic acids, vectors, and cells. These mixtures and/or compositions, may optionally include adjuvants.

The invention further provides a method of inducing an immune response in an animal directed against:
(i) a CEA protein or fragment thereof; and/or
(ii) a CEA agonist polypeptide/protein of the invention; and/or
(iii) a CEA epitope; and/or
(iv) a modified CEA epitope; and/or
(v) cells expressing a CEA protein or fragment thereof, CEA agonist polypeptide/protein of the invention, CEA epitope, modified CEA epitope; and/or;
(vi) cells binding a CEA protein or fragment thereof, CEA agonist polypeptide/protein of the invention, CEA epitope, modified CEA epitope,
comprising administering to said animal a CEA agonist polypeptide/protein, nucleic acid, vector, cell, or mixture and/or composition of the invention in an amount sufficient to induce an immune response.

The invention further contemplates a method of inhibiting a CEA epitope expressing carcinoma cell in a patient comprising administering to said patient an effective amount of a CEA agonist polypeptide/protein, nucleic acid, vector, cell, or mixture and/or composition of the invention.

The invention in yet a further aspect provides for a treatment for cancer comprising any one of the aforementioned methods for inducing immune responses and/or inhibiting carcinoma cells expressing a CEA epitope.

Other features and advantages of the present invention will become apparent from the following detailed description. It should be understood, however, that the detailed description and the specific examples while indicating particular embodiments of the invention are given by way of illustration only, since various changes and modifications within the spirit and scope of the invention will become apparent to those skilled in the art from this detailed, description.

### BRIEF DESCRIPTION OF THE FIGURES

The present invention will be further understood from the following description with reference to the figures, in which:
Figure 1 depicts the nucleic acid and amino acid sequence of an embodiment of the invention encompassing modified CEA.
Figure 2 depicts 3 schematic representations encompassing the genomic structure of particular recombinant fowlpox, vaccinia and MVA constructs expressing modified CEA.
Figure 3 depicts a schematic representation of the *Xhol* restriction map profile of an ALVAC(2)-CEA (modified)/human B7.1 construct.
Figure 4 depicts the nucleic acid sequence of the H6-promoted CEA (modified)/human B7.1 insertion cassette used in the production of the construct of Figure 3.
Figure 5 depicts the results of an immunoprecipitation analysis of HeLa cells infected with various ALVAC recombinant constructs.
Figure 6 depicts the results of a western blot analysis of HeLa cells infected with various ALVAC recombinant constructs.

### DETAILED DESCRIPTION OF THE INVENTION

The invention discloses CEA agonist polypeptides/proteins comprising a modified CEA epitope wherein said modified CEA epitope comprises the sequence YLSGADLNL, nucleic acids coding therefor, vectors and/or cells comprising said nucleic acids (collectively designated as "agents" of the invention), and mixtures/compositions of the aforementioned. All of the aforementioned agents and mixtures/compositions of the invention have the ability to induce or elicit an immune response against a CEA protein fragment thereof, a CEA agonist polypeptide/protein of the invention, a CEA epitope and/or modified CEA epitope, and/or cells binding or expressing the aforementioned. An "immune response" is defined as any response of the immune system, for example, of either a cell-mediated (i.e. cytotoxic T-lymphocyte mediated) or humoral (i.e. antibody mediated) nature. As is known to those skilled in the art, various assays/methodologies exist for the assessment and/or monitoring of immunological responses.

Within the context of cell-mediated immune responses, tumor associated antigen proteins (such as CEA) are processed by intracellular proteases into smaller epitope peptides which are subsequently transported to the cell surface tightly bound in a cleft on an MHC HLA class I molecule. T cells recognize these small epitope peptides only when presented in association with MHC HLA Class I molecules on the surface of appropriate cells. Analogously, in the context of humoral immune responses proteins can be processed into smaller epitope peptides which are subsequently presented on cell surfaces (i.e. antigen presenting cells) in association with MHC HLA class II molecules. Said complexes are recognized by appropriate cells of the humoral immune system.

As is well known to those skilled in the art, short peptides (i.e. epitopes) composed of amino acid sequences of about 8 to 12 amino acids derived from antigens are capable of binding directly within the cleft of an HLA class I molecule without intracellular processing. As previously noted, a number of such epitope peptides derived from CEA have been identified. Moreover, some of these CEA-specific antigen epitopes have demonstrated the capacity to induce/elicit immune responses wherein appropriate CEA expressing target cells are lysed. The CEA agonist polypeptides/proteins of the present invention (comprising a modified CEA epitope) elicit an improved immune' response (hence, deemed to be CEA "agonist(s)") by comparison to that observed when normal/unmodified CEA (comprising normal epitopes) is employed as an immunogen.

As encompassed by this invention, the CEA agonist polypeptides/proteins comprise a modified epitope containing the amino acid sequence YLSGADLNL (designated "CAP16D"). The counterpart sequence of the naturally occurring epitope in unmodified CEA is YLSGANLNL (designated "CAP1"; Zaremba, S. et al. (1997) Cancer Res. 57:4570). In a particular embodiment of the invention, the CEA agonist polypeptide/protein has the amino acid sequence of SEQ ID NO: 1 (Figure 1). It is further recognized that CEA agonist polypeptide(s)/protein(s) embodiments of the invention encompass both precursor and mature forms of polypeptide/protein (for example, see Oikawa, S. et al. (1987) *Biochem. Biophys. Res. Commun.* 142:51 1-518).

The CEA agonist polypeptides/proteins of the invention may be prepared using a variety of methods known to one skilled in the art. Accordingly, recombinant DNA methods known to those skilled in the art can be utilized to provide these polypeptides. Nucleic acid sequences which encode for the CEA agonist polypeptides/proteins of the invention may be incorporated in a known manner into appropriate expression vectors (i.e. recombinant expression vectors). Possible expression vectors include (but are not limited to) cosmids, plasmids, or modified viruses (e.g. replication defective retroviruses, adenoviruses and adeno-associated viruses, lentiviruses, herpes viruses, poxviruses), so long as the vector is compatible with the host cell used. The expression "vector is compatible with the host cell" is defined as contemplating that the expression vector(s) contain a nucleic acid molecule of the invention (hereinafter described) and attendant regulatory sequence(s) selected on the basis of the host cell(s) to be used for expression, said regulatory sequence(s) being operatively linked to the nucleic acid molecule. "Operatively linked" is intended to mean that the nucleic acid is linked to regulatory sequence(s) in a manner which allows expression of the nucleic acid. Suitable regulatory sequences may be derived from a variety of sources, including bacteria), fungal, or viral genes. (Forexample, see the regulatory sequences described in Goeddel, Gene Expression Technology: Methods in Enzymology 185, Academic Press, San Diego, CA (1990). Selection of appropriate regulatory sequence(s) is dependent on the host cell(s) chosen, and may be readily accomplished by one of ordinary skill in the art. Examples of such regulatory sequences include the following: a transcriptional promoter and enhancer, RNA polymerase binding sequence, or a ribosomal binding sequence (including a translation initiation signal). Depending on the host cell chosen and the expression vector employed, other additional sequences (such as an origin of replication, additional DNA restriction sites, enhancers, and sequences confeffing inducibility of transcription) may be incorporated into the expression vector.

The aforementioned expression vectors of the invention may also contain a selectable marker gene which facilitates the selection of host cells transformed or transfected with a recombinant molecule of the invention. Examples of selectable marker genes are genes encoding a protein such as G418 and hygromycin (which confer resistance to certain drugs), β-galactosidase, chloramphenicol acetyltransferase, or firefly luciferase.

Transcription of the selectable marker gene is monitored by changes in the concentration of the selectable marker protein such as β-galactosidase, chloramphenicol acetyltransferase, or firefly luciferase. Transformant cells can be selected with appropriate selection molecules if the selectable marker gene encodes a protein conferring antibiotic resistance (i.e. G418 in context of neomycin resistance). As is known to one skilled in the art, cells that have incorporated the selectable marker gene will survive, while cells which do not have any such incorporated detectable marker will die. This makes it possible to visualize and assay for expression from recombinant expression vectors of the invention. It will also be appreciated that selectable markers can be introduced on a separate vector from the nucleic acid of interest.

The recombinant expression vectors may also contain genes which encode a fusion moiety which provides increased expression of the polypeptides of the invention; increased solubility of the polypeptides of the invention; and/or aids in the purification of a target recombinant protein by acting as a ligand in affinity purification. For example, a proteolytic cleavage site may be added to the target recombinant polypeptide to allow separation of the recombinant polypeptide peptide(s) from the fusion moiety subsequent to purification of the fusion protein.

The CEA agonist polypeptides/proteins of the invention may also be prepared by chemical synthesis using techniques well known in the chemistry of proteins such as solid phase synthesis (Merrifield (1964) *J. Am. Chem. Assoc*. 65:2149) or synthesis in homogenous solution (Methods of Organic Chemistry, E. Wansch (Ed.) Vol. 15, pts. I and II, Thieme, Stuttgart (1987)).

Additional embodiments of the invention encompass nucleic acids coding for the CEA agonist polypeptides/proteins hereinbefore described. As defined herein, "nucleic acid(s)" encompass (but is not limited to) viral nucleic acid(s), plasmid(s), bacterial DNA, naked/free DNA and RNA. The nucleic acids encompass both single and double stranded forms. As such, these nucleic acids comprise the relevant base sequences coding for the aforementioned polypeptides. For purposes of definitiveness, the "relevant base sequence's coding for the aforementioned polypeptides" further encompass complementary nucleic acid sequences.

In one embodiment of the invention, the nucleic acid has the sequence denoted by SEQ ID NO:2 (Figure 1). In further embodiments of the invention, the nucleic acid comprises this sequence (i.e. SEQ ID NO:2 (Figure 1)). It is further recognized that additional embodiments of the invention may consist of and/or comprise nucleic acid sequences coding for precursor or mature CEA agonist polypeptide(s)/protein(s) (for example, see Oikawa, S. et al. (1987) *Supra)*.

Bacterial DNA useful in embodiments of the invention are known to those of ordinary skill in the art. These bacteria include, for example, *Shigella, Salmonella, Vibrio cholerae, Lactobacillus, Bacille Calmette Guérin (BCG),* and *Streptococcus.* In bacterial DNA embodiments of the invention, nucleic acid of the invention may be inserted into the bacterial génome, can remain in a free state, or be parried on a plasmid.

Viral nucleic acid embodiments of the invention may be derived from a poxvirus or other virus such as adenovirus or alphavirus. Preferably the viral nucleic acid is incapable of integration in recipient animal cells. The elements for expression from said nucleic acid may include a promoter suitable for expression in recipient animal cells.

Embodiments of the invention encompass poxviral nucleic acid selected from the group consisting of avipox, orthopox, and suipox nucleic acid. Particular embodiments encompass poxviral nucleic acid selected from vaccinia, fowlpox, canary pox and swinepox; specific examples include TROVAC, NYVAC, ALVAC, MVA, Wyeth and Poxvac-TC (described in more detail below).

It is further contemplated that nucleic acids of this invention may further comprise nucleic acid sequences, encoding at least one member chosen from the group consisting cytokines, lymphokines, and co-stimulatory molecules. Examples include (but are hot limited to) interleukin 2, interleukin 12, interleukin 6, interferon gamma, tumor necrosis factor Alpha, GM-CSF, B7.1, B7.2, ICAM-1, LFA-3, and Cd72.

Standard techniques of molecular biology for preparing and purifying nucleic acids well known to those skilled in the art can be used in the preparation of aspects of the invention (for example, as taught in *Current Protocols in Molecular Biology,* F.M. Ausubel et al. (Eds.), John Wiley and Sons, Inc, N.Y., U.S.A. (1998), Chpts. 1, 2 and 4; *Molecular Cloning: A Laboratory Manual (2nd Ed.),* J. Sambrook, E.F. Fritsch and T. Maniatis (Eds.), Cold Spring Harbor Laboratory Press, N.Y., U.S.A. (1989), Chpts. 1, 2, 3 and 7).

Aspects of this invention further encompass vector comprising, the aforementioned nucleic acids. In certain embodiments, said vectors may be recombinant viruses or bacteria.

Adenovirus vectors and methods, for their construction have been described (e.g. U.S. Patent Nos. 5994132, 5932210, 6057158 and Published PCT Applications WO 9817783, WO 9744475, WO 9961034, WO 9950292, WO 9927101, WO 9720575, WO 9640955, WO 9639534, all of which are herein incorporated by reference). Alphavirus vectors have also been described in the art and can be used in embodiments of this invention (e.g. U.S. Patent Nos. 5792462, 5739026, 5843723, 5789245, and Published PCT Applications WO 9210578, WO 9527044, WO 9531565, WO 9815636 all of which are herein incorporated by reference), as have lentivirus vectors (e.g. U.S. Patent Nos. 6013516, 5994136 and Published PCT Applications WO 9617816, WO 9712622, WO 9817815, WO 9839463, WO 9846083, WO 9915641, WO 9919501, WO 9930742, WO 9931251, WO 9851810, WO 0000600 all of which are herein incorporated by reference). Poxvirus vectors that can be used include, for example, avipox, orthopox or suipox poxvirus (as described in U.S. Patent Nos. 5364773, 4603112, 5762938, 5378457, 5494807, 5505941, 5756103, 5833975 and 5990091 all of which are herein incorporated by reference). Poxvirus vectors comprising a nucleic acid coding for a CEA agonist polypeptide/protein of the invention can be obtained by homologous recombination as is known to one skilled in the art, as such, the nucleic acid coding for the CEA agonist polypeptide/protein is inserted into the viral genome under appropriate conditions for expression in mammalian cells (as described below).

In one embodiment of the invention the poxvirus vector is ALVAC (1) or ALVAC (2) (both of which have been derived from canarypox virus). ALVAC (1) (or ALVAC (2)) does not productively replicate in non-avian hosts, a characteristic thought to improve its safety profile. ALVAC (1) is an attenuated canarypox virus-based vector that was a plaque-cloned derivative of the licensed canarypox vaccine, Kanapox (Tartaglia et al. (1992) *Virology* 188:217; U.S. Patent Nos. 5505941, 5756103 and 5833975 all of which are incorporated herein by reference). ALVAC (1) has some general properties which are the same as some general properties of Kanapox. ALVAC-based recombinant viruses expressing extrinsic immunogens have also been demonstrated efficacious as vaccine vectors (Tartaglla et al, In AIDS Research Reviews (vol. 3) Koff W., Wong-Staol F. and Kenedy R.C. (eds.), Marcel Dekker NY, pp. 361-378 (1993a); Tartaglia, J. et al. (1993b) *J. Vivol*. 67:2370). For instance, mice immunized with an ALVAC (1) recombinant expressing the rabies virus glypoprotein were protected from lethal challenge with rabies virus (Tartaglia, J. et at, (1992) *supra)* demonstrating the potential for ALVAC (1) as a vaccine vector. ALVAC-based recombinants have also proven efficacious in dogs challenged with canine distemper virus (Taylor, J. et al. (1992) *Virology* 187:321) and rabies virus (Perkus, M.E. et al., In Combined Vaccines and Simultaneous Administration: Current Issues and Perspective, Annals of the New York Academy of Sciences (1994)), in cats challenged with feline leukemia virus (Tartaglia, J. et al., (1993b) *supra),* and in horses challenged with equine influenza virus (Taylor, J. et aI., In Proceedings of the Third International Symposium on Avian Influenza, Univ. of Wisconsin-Madison, Madison, Wisconsin, pp. 331-335 (1993)).

ALVAC (2) is a second-generation ALVAC vector in which vaccinia transcription elements E3L and K3L have been inserted within the C6 locus (U.S. Patent No. 5990091, incorporated herein by reference). The E3L encodes a protein capable of specifically binding to dsRNA. The K3L ORF has significant homology to E1 F-2. Within ALVAC (2) the E3L gene is under the transcriptional control of its natural promoter, whereas K3L has been placed under the control of the early/late vaccine H6 promoter. The E3L and K3L genes act to inhibit PKR activity in cells infected with ALVAC (2), allowing enhancement of the level and persistence of foreign gene expression.

Additional viral vectors encompass natural host-restricted poxviruses. Fowlpox virus (FPV) is the prototypic virus of the Avipox genus of the Poxvirus family. Replication of avipox viruses is limited to avian species (Matthews, R.E.F. (1982) *Intervirology* 17:42) and there are no reports in the literature of avipox virus causing a productive infection in any non-avian species including man. This host restriction provides an inherent safety barrier to transmission of the virus to other species and makes use of avipox virus based vectors in veterinary and human applications an attractive proposition.

FPV has been used advantageously as a vector expressing immunogens from poultry pathogens. The hemagglutinin protein of a virulent avian influenza virus was expressed in an FPV recombinant. After inoculation of the recombinant into chickens and turkeys, an immune response was induced which was protective against either a homologous or a heterologous virulent influenza virus challenge (Taylor, J. et al. (1968) Vaccine 6: 504). FPV recombinants expressing the surface glycoproteins of Newcastle Disease Virus have also been developed (Taylor, J. et al. (1990) *J. Virol*. 64:1441; Edbauer, C. et al. (1990) Virology 179:901); U.S. Patent No. 5766599 incorporated herein by reference).

A highly attenuated strain of vaccinia, designated MVA, has also been used as a vector for poxvirus-based vaccines. Use of MVA is described in U.S. Patent No. 5,185,146.

Other attenuated poxvirus vectors have been prepared via genetic modification to wild type strains of vaccinia. The NYVAC vector, for example, is derived by deletion of specific virulence and host-range genes from the Copenhagen strain of vaccinia (Tartaglia, J. et al. (1992), *supra;* U.S. Patent Nos. 5364773 and 5494807 incorporated herein by reference) and has proven useful as a recombinant vector in eliciting a protective immune response against expressed foreign antigens.

Recombinant viruses can be constructed by processes known to those skilled in the art (for example, as previously described for vaccinia and avipox viruses; U.S. Patent Nos. 4769330; 4722648; 4603112; 5110587; and 5174993-all of which are incorporated herein by reference).

In further embodiments of the invention, live and/or attenuated bacteria may also be used as vectors. For example, non-toxicogenic *Vibrio cholerae* mutant strains may be useful as bacterial vectors in embodiments of this invention; as described in US Patent, No. 4,882,278 (disclosing a strain in which a substantial amount of the coding sequence of each of the two ctxA alleles has been deleted so that no functional cholera toxin is produced), WO 92111354 (strain in which the irgA locus is inactivated by mutation; this mutation can be combined in a single strain with ctxA mutations), and WO 94/1533 (deletion mutant lacking. functional ctxA and attRS1 DNA sequences). These strains can be genetically engineered to express heterologous antigens, as described in WO 94/19482. (All of the aforementioned issued patent/patent applications are incorporated herein by reference). Attenuated *Salmonella typhimurium* strains, genetically engineered for recombinant expression of heterologous antigens and their use as oral immunogens are described, for example, in WO 92/11361.

As noted, those skilled in the art will readily recognize that other bacterial strains useful as bacterial vectors in embodiments of this invention include (but are not limited to) *Shigella flexneri, Streptococcus gordonii,* and *Bacille Calmette Guerin* (as described in WO 88/6626, WO 90/0594, WO 91/13157, WO 92/1796, and WO 92/21376; all of which are incorporated herein by reference). In bacterial vector embodiments of this invention, a nucleic acid coding for a CEA agonist polypeptide/protein may be inserted into the bacterial genome, can remain in a free state, or be carried on a plasmid.

It is further contemplated that the invention encompasses vectors which comprise nucleic acids coding for at least one member from the group consisting of cytokines, lymphokines and immunostimulatory molecules. Said nucleic acid sequences can be contiguous with sequences coding for CEA agonist polypeptide/proteins, or encoded on distinct nucleic acids.

Cells comprising the aforementioned nucleic acids coding for CEA agonist polypeptides/proteins encompass further embodiments of the invention. These cells encompass any potential cell into which the aforementioned nucleic acid might be introduced and/or transfected (for example, bacteria, COS cells, Vero cells, chick embryo fibroblasts, tumor cells, and antigen presenting cells). The choice of process for the introduction and/or transfection into cells is dependant upon the intrinsic nature of the nucleic acid (i.e. free DNA, plasmid, incorporated into a recombinant virus), as will be known to one skilled in the art (for example, as taught in *Current Protocols in Molecular Biology,* F.M. Ausubel et al. (Eds.), John Wiley and Sons, Inc., N.Y., U.S.A. (1998), Chpt. 9; *Molecular Cloning: A Laboratory Manual (2nd Ed.),* J. Sambrook, E.F. Fritsch and T. Maniatis (Eds.), Cold Spring Harbor Laboratory Press, *N*.*Y*., U.S.A. (1989), Chpt's. 1,2, 3 and 16).

It is well documented that the class I and class II proteins of the major histocompatibility complex (MHC) perform a central immunological function in focusing T-lymphocytes of the immune system (i.e. CD8+ and CD4+ T lymphocytes). MHC class I proteins are expressed in nearly all nucleated cell types throughout the human body; MHC class II molecules are expressed mainly on antigen-presenting cells (APCs; namely, mononuclear phagocytes, Langerhans-dendritic cells, and B lymphocytes). These distinct classes of cell surface molecules (i.e. class I and class II) present peptides/epitopes (derived from intracellular processing of protein antigens) to T lymphocytes (CD8+ and CD4+ T lymphocytes respectively) thus initiating both cellular and humoral immune responses. Generally, epitopes/peptides derived from alloantigens, tumor antigens or viruses will be presented in association, with MHC class I molecules; extracellular antigens/proteins will be presented in association with MHC class II molecules. However, in some contexts endogenous antigens can also be presented in association with MHC class II molecules. [These general immunological principles are well described in the art-as, for example, in *Encyclopedia of Immunology (2nd Ed.),* Peter J. Delves (Ed.-in-Chief), Academic Press, San Diego, U.S.A., pp. 174-8, 191-8, 1108-13, 1690-709(1998).]

As such, embodiments of the invention contemplate cells into which has been introduced/transfected a nucleic acid coding for a CEA agonist polypeptide/protein wherein said cells express said polypeptide/protein.

As further conceived herein, embodiments of the invention also encompass cells into which has been introduced/transfected a nucleic acid coding for CEA agonist polypeptide/protein wherein said cells also express a MHC HLA molecule (i.e. class I and/or class II). In further embodiments, these cells are antigen-presenting cells, possibly selected from the group consisting of mononuclear phagocytes, Langerhans dendritic cells ("dendritic cell(s)"), and B lymphocytes.

Aspects of this invention contemplate mixtures of the CEA agonist polypeptide(s)/protein(s), nucleic acids coding therefor, vectors comprising said nucleic acids, or cells comprising said nucleic acids, and at least one members selected from the group consisting of cytokines, lymphokines, immunostimulatory molecules, and nucleic acids coding therefor. Additional embodiments of this invention further encompass pharmaceutical compositions comprising the CEA agonist polypeptide/protein, nucleic acids coding therefor, vectors cells or mixtures for administration to subjects in a biologically compatible form suitable for administration *in vivo*. By "biologically compatible form suitable for administration *in vivo"* is meant a form of the substance to be administered in which any toxic effects are outweighed by the therapeutic effects. Administration of a therapeutically active amount of the pharmaceutical compositions of the present invention, or an "effective amount", is defined as an amount effective at dosages and for periods of time, necessary to achieve the desired result of eliciting an immune response in a human. A therapeutically effective amount of a substance may vary according to factors such as the disease state/health, age, sex, and weight of the recipient, and the inherent ability of the particular polypeptide, nucleic acid coding therefor, or recombinant virus to elicit a desired immune response. Dosage regima may be adjusted to provide the optimum therapeutic response. For example, several divided doses may be administered daily or on at periodic intervals, and/or the dose may be proportionally reduced as indicated by the exigencies of the therapeutic situation.

The compositions described herein can be prepared by *per* se known methods for the preparation of pharmaceutically acceptable compositions which can be administered to subjects, such that an effective quantity of the active substance (i.e. CEA agonist polypeptide/protein, nucleic acid coding therefor) is combined in a mixture with a pharmaceutically acceptable vehicle. Suitable vehicles are described, for example, in "Handbook of Pharmaceutical Additives" (compiled by Michael and Irene Ash, Gower Publishing Limited, Aldershot, England (1995)). On this basis, the compositions include, albeit not exclusively, solutions of the substances in association with one or more pharmaceutically acceptable vehicles or diluents, and may be contained in buffered solutions with a suitable pH and/or be iso-osmotic with physiological fluids. In this regard, reference can be made to U.S. Patent No. 5,843,456. These compositions may further comprise an adjuvant (as described below).

Methods of inducing or eliciting an immune response in an animal directed against:
a CEA protein or fragment thereof; and/or
a CEA agonist polypeptide/protein of the invention; and/or
a CEA epitope; and/or
a modified CEA epitope; and/or
cells expressing a CEA protein or fragment thereof, CEA agonist polypeptide/protein of the invention, CEA epitope, modified CEA epitope; and/or cells binding a CEA protein or fragment thereof, CEA agonist polypeptide/protein of the invention, CEA epitope, modified CEA epitope, comprising the step of; administering to said animal a CEA agonist polypeptide/protein or fragment thereof, a nucleic acid coding therefor, a vector or cell comprising said nucleic acid; mixtures thereof or pharmaceutical compositions of the aforementioned (hereinafter collectively referred to as "immunizing agent's)", "agent's)', or "immunogen(s)") are also within the scope of this invention. As previously noted, an "immune response" is defined as any response of the immune system, for example, of either a cell-mediated (i.e. cytotoxic T-lymphocyte mediated) or humoral (i.e. antibody mediated) nature. These immuhe responses can be assessed by a number of *in vivo* or *in vitro* assays well known to one skilled in the art (for example, (but not limited to) antigen specific cytotoxicity assays, production of cytotoxins, regression of tumors expressing CEA/CEA-epitopes, inhibition of cancer cells expressing CEA/CEA epitopes).

Further embodiments of the invention encompass methods inhibiting a CEA epitope expressing carcinoma cell, in a patient comprising administering to said patient an effective amount of an immunogen of the invention. Patients with solid tumors expressing CEA (or epitopes thereof) include (but are not limited to) those suffering from colon cancer, lung cancer, pancreas cancer, endometrial cancer, breast cancer, thyroid cancer, melanoma, oral cancer, laryngeal cancer, seminoma, hepatocellular cancer, bile duct cancer, squamous cell carcinoma, and prostate cancer. As such, methods of treating patients with cancer per se encompassing the aforementioned methods of inducing an immune response and/or inhibiting a CEA epitope expressing carcinoma cell are contemplated aspects/embodiments of the invention.

As known to one of ordinary skill in the art, an animal may be immunized with an immunogen of the invention by any conventional route. This may include, for example, immunization via a mucosal surface (e.g., ocular, intranasal, oral, gastric, pulmonary, intestinal, rectal, vaginal, or urinary tract) or via a parenteral route (e.g., subcutaneous, intradermal, intramuscular, intravenous, or intraperitoneal). Preferred routes depend upon the choice of the immunogen (i.e. polypeptide vs. nucleic acid, recombinant/virus, composition formulation, etc.). The administration can be achieved in a single dose or repeated at intervals. The appropriate dosage is dependant on various parameters understood by the skilled artisans, such as the immunogen itself (i.e. polypeptide vs. nucleic acid (and more specifically type thereof)), the route of administration and the condition of the animal to be vaccinated (weight, age and the like). As such, embodiments of this invention encompass methods of eliciting immune responses in animals comprising administering an effective amount of a CEA agonist polypeptide/proteins of the invention, a nucleic acid coding therefore, vector or cells or recombinant virus comprising said nucleic acid, mixtures thereof, or pharmaceutical compositions of the aforementioned.

As previously noted, nucleic acids (in particular plasmids and/or free/naked DNA and/or RNA coding for the CEA agonist polypeptide/protein of the invention) can be administered to an animal for purposes of inducing/eliciting an immune response (for example, U.S. Patent No. 5589466; McDonnell and Askari, NEJM 334:42-45 (1996), Kowalczyk and Ertl, *Cell Mot Life* Sct 55:751-770 (1999)). Typically, this nucleic acid is a form that is unable to replicate in the target animal's cell and unable to 'integrate in said animal's genome. The DNA/RNA molecule encoding the CEA agonist polypeptide/protein is also typically placed under the control of a promoter suitable for expression in the animal's cell. The promoter can function ubiquitously or tissue specifically. Examples of non-tissue specific promoters include the early Cytomegalovirus (CMV) promoter (described in U.S. Patent No. 4,168,062) and the Rous Sarcoma Virus promoter. The desmin promoter is tissue-specific and drives expression in muscle cells. More generally, useful vectors have been described (i.e., WO 94/21 797).

For administration of nucleic acids coding for a CEA agonist polypeptide/protein of the invention, said nucleic acid can encode a precursor or mature form of the polypeptide/protein. When it encodes a precursor form, the precursor form can be homologous (for example, see Oikawa, S. et al. (1987) *Supra)* or heterologous. In the latter case, a eucaryotic leader sequence can be used, such as the leader sequence of the tissue-type plasminogen factor (tPA).

For use as an immunogen, a nucleic acid of the invention can be formulated according to various methods known to a skilled artisan. First, a nucleic acid can be used in a naked/free form, free of any delivery vehicles (such as anionic liposomes, cationic lipids, microparticles, (e.g., gold microparticles), precipitating agents (e.g., calcium phosphate) or any other transfection-facilitating agent. In this case the nucleic acid can be simply diluted in a physiologically acceptable solution (such as sterile saline or sterile buffered saline) with or without a carrier. When present, the carrier preferably is isotonic, hypotonic, or weakly hypertonic, and has a relatively low ionic strength (such as provided by a sucrose solution (e.g., a solution containing 20% sucrose)).

Alternatively, a nucleic acid can be associated with agents that assist in cellular uptake. It can be, i.e., (i) complemented with a chemical agent that modifies the cellular permeability (such as bupivacaine; see, for example, WO 94/16737), (ii) encapsulated into liposomes, or (iii) associated with cationic lipids or silica, gold, or tungsten microparticles.

Cationic lipids are well known in the art and are commonly used for gene delivery. Such lipids include Lipofectin (also known as DOTMA (N-[1-(2,3-dioleyloxy)propyl]-N,N,N-trimethylammonium chloride), DOTAP (1 ,2-bis(oleyloxy)-3-(trimethylammonio)propane). DDAB (dimethyldioctadecylammonium bromide), DOGS (dioctadecylàmidologlycyl spermine) and cholesterol derivatives such as DC-Chol (3 beta-(N-(N',N'-dimethyl aminomethane)-carbarnoyl) cholesterol). A description of these cationic lipids can be found in EP 187,702, WO 90/11092, U.S. Patent No. 5,283,185, WO 91/15501, WO 95/26356, and U.S. Patent No. 5,527,928. Cationic lipids for gene delivery are preferably used in association with a neutral lipid such as DOPE (dioleyl phosphatidylethanolamine), as for example, described in WO 90/11092.

Other transfection-facilitating compounds can be added to a formulation cdntaining cationic liposomes. A number of them are described in, for example, WO 93/18759, WO 93/19768, WO 94/25608, and WO 95/2397. They include, i.e., Spermine derivatives useful for facilitating the transport of DNA through the nuclear membrane (see, for example, WO 93/18759) and membrane-permeabilizing compounds such as GALA, Gramicidine 5, and cationic bile salts (see, for example, WO 93/19768).

Gold or tungsten microparticles can also be used for gene delivery (as described in WO 91/359 and WO 93/17706). In this case, the microparticle-coated polynucleotides can be injected via intradermal or intraepidermal routes using a needleless injection device ("gene gun"), such as those described, for example, in U.S. Patent No. 4,945,b50, U.S. Patent No. 5,015,580, and WO 94/24263.

Anionic and neutral liposomes are also well-known in the art (see, for example, Liposomes: A Practical Approach, RPC New Ed, IRL Press (1990), for a detailed description of methods for making liposomes) and are useful for delivering a large range of products, including nucleic acids.

Particular embodiments of the aforementioned methods (i.e. to induce/elicit immune responses and/or to inhibit a CEA epitope expressing carcinoma cell in a patient) encompass prime-boost protocols for the administration of immunogens of the invention. More specifically, these protocols encompass (but are not limited to) a "priming" step with a particular/distinct form of immunogen (i.e. nucleic acid (for example, plasmid, bacterial/viral/free or naked) coding for an immunogen, or vector (i.e. recombinant virus, bacteria) comprising said nucleic acid) followed by at least one "boosting" step encompassing the administration of an alternate (i.e. distinct from that used to "prime") form of the immunogen (i.e. protein or fragment thereof (for example, epitope-peptide), nucleic acid coding for an immunogen (or fragment thereof), or vector comprising said nucleic acid). Examples of "prime-boost" methodologies are known to those skilled in the art (as taught, for example, in PCT published applications WO 00/00216, WO 98/58956, WO 98/56919, WO 97/39771). One advantage of said protocols is the potential to circumvent the problem of generating neutralizing immune responses to viral vectors *per* se wherein is inserted incorporated nucleic acids encoding the immunogen or fragments thereof (see for example, R.M. Conty et al. (2000) *Clin. Cancer* Res. 6:34-41).

As is well known to those of ordinary skill in the art, the ability of an immunogen to induce/elicit an immune response can be improved if, regardless of administration formulation (i.e. recombinant virus, nucleic acid, polypeptide), said immunogen is coadministered with an adjuvant. Adjuvants are described and discussed in "Vaccine Design-the Subunit and Adjuvant Approach" (edited by Powell and Newman, 'Plenum Press, New York, U.S.A., pp. 61-79 and 141-228 (1995)). Adjuvants typically enhance the immunogenicity of an Immunogen but are not necessarily immunogenic in and of themselves. Adjuvants may act by retaining the immunogen locally near the site of administration to produce a depot effect facilitating a slow, sustained release of immunizing agent to cells of the immune system. Adjuvants can also attract cells of the, immune system to an immunogen depot and stimulate such cells to elicit immune responses. As such, embodiments of this invention encompass compositions further comprising adjuvants.

Desirable characteristics of ideal adjuvants include:
1) lack of toxicity:
2) ability to stimulate a long-lasting immune response;
3) simplicity of manufacture and stability in long-term storage;
4) ability to elicit both cellular and humoral responses to antigens administered by various routes, if required:
5) synergy with other adjuvants;
6) capability of selectively interacting with populations of antigen presenting cells (APC);
7) ability to specifically elicit appropriate TRI or TH2 cell-specifib immune responses; and
8) ability to selectively increase appropriate antibody isotype levels (for example, IgA) against antigens/immunogens.

However, many adjuvants are toxic and can cause undesirable side effects, thus making them unsuitable for use in humans and many animals. For example, some adjuvants may induce granulomas, acute and chronic inflammations (i.e. Freund's complete adjuvant (FCA)), cytolysis (i.e. saponins and pluronic polymers) and pyrogenicity, arthritis and anterior uveitis (i.e. muramyl dipeptide (MDP) and lipopolysaccharide (LPS)). Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established. Notwithstanding, it does have limitations. For example, alum is ineffective for influenza vaccination and inconsistently elicits a cell mediated immune response with other immunogens. The antibodies elicited by alum-adjuvanted antigens are mainly of the IgG1 isotype in the mouse, which may not be optimal for protection in vaccination contexts.

Adjuvants may be characterized as "intrinsic" or "extrinsic". Intrinsic adjuvants (such as lipopolysaccharides) are integral and normal components of agents which in themselves are used as vaccines (i.e. killed or attenuated bacteria), Extrinsic adjuvants are typically nonintegral immunomodulators generally linked to antigens in a noncovalent manner, and are formulated to enhance the host immune response.

In embodiments of the invention, adjuvants can be at least one member chosen from the group consisting of cytokines, lymphokines, and co-stimulatory molecules. Examples include (but are not limited to) interleukin 2, interleukin 12, interleukin 6, interferon gamma, tumor necrosis factor alpha, GM-CSF, 87.1, 87.2, ICAM-1, LFA-3, and CD72. Particular embodiments specifically encompass the use of GM-CSF as an adjuvant (as taught, for example, in U.S. Patent Nos. 5679356, 5904920, 5637483, 5759535, 5254534, European Patent Application EP 211684, and published PCT document WO 97/28816 all of which are herein incorporated by reference).

A variety of potent extrinsic adjuvants have been described. These include (but are not limited to) saponins complexed to membrane protein antigens (immune stimulating complexes), pluronic polymers with mineral oil, killed mycobacteria and mineral oil, Freund's complete adjuvant, bacterial products such as muramyl dipeptide (MDP) and lipopolysaccharide (LPS), as well as lipid A, and liposomes.

The use of saponins *per* se as adjuvants is also well known (Lacaille-Dubois, M. and Wagner, H (1996) *Phytomedicine* 2:363). For example, Quil A (derived from the bark of the South American tree, Quillaja Saponaria Molina) and fractions thereof has been extensively described (i.e. U.S. Patent No. 5057540; Kensil, C.R. (1996) *Crit* Rev *Ther Drug Carrier Syst* 12:1; and European Patent EP 362279). The haemolytic saponins Q521 and QSI7 (HPLC purified fractions of Quil A) have been described as potent systemic adjuvants (U.S. Patent No. 5057540: European Patent EP 362279). Also described in these references is the use of QS7 (a non-haemolytic fraction of Quil-A) which acts as a potent adjuvant for systemic vaccines. Use of QS2I is further described in Kensil et al. ((1991) *J*. *Immunol* 146:431). Combinations of O521 and polysorbate or cyclodextrin are also known (WO 9910008). Particulate adjuvant systems comprising fractions of Quil A (such as QS2I and QS7) are described in WO 9633739 and WO 9611711.

Another preferred adjuvant/immunostimulant is an immunostimulatory oligonucleotide containing unmethylated CpG dinucleotides ("CpG"). CpG is an abbreviation for cytosine-guanosine dinucleotide motifs present in DNA. CpG is known in the art as being an adjuvant when administered by both systemic and mucosal routes (WO 9602555; European Patent EP 468520; Davies et al. (1998). *J. Immunol*. 160:87; McCluskie and Davis (1998) *J. Immunol* 161:4463). In a number of studies, synthetic oligonucleotides derived from BCG gene sequences have also been shown to be capable of inducing immunostimulatory effects (both *in vitro* and *in vivo;* Krieg, (1995) *Nature* 374:546). Detailed analyses of immunostimulatory oligonucleotide sequences has demonstrated that the CG motif must be in a certain sequence context, and that such sequences are common in bacterial DNA but are rare in vertebrate DNA. (For example, the immunostimulatory sequence is often: purine, purine, C, G, pyrimidine, pyrimidine, wherein the CG motif is not methylated; however other unmethylated CpG sequences are known to be immunostimulatory and as such may also be used in the present invention.) As will be evident to one of normal skill in the art, said CG motifs/sequences can be. incorporated into nucleic acids of the invention *per se,* or reside on distinct nucleic acids.

A variety of other adjuvants are taught in the art, and as such are encompassed by embodiments of this invention. U.S. Patent No. 4,855,283 granted to Lockhoff et al. (incorporated herein by reference) teaches glycolipid analogues and their use as adjuvants. These include N-glycosylamides, N-glycosylureas and N-glycosylcarbamates, each of which is substituted in the sugar residue by an amino acid, as immuno-modulators or adjuvants. Furthermore, Lockhoff et al. ((1991) *Chem. mt. Ed. EngI*. 30:1611) have reported that N-glycolipid analogs displaying structural similarities to the naturally-occurring glycolipids (such as glycophospholipids and glycoglycerolipids) are also capable of eliciting strong immune responses in both herpes simplex virus vaccine and pseudorabies virus vaccine.

U.S. Patent No. 4,258,029 granted to Moloney (incorporated herein by reference) teaches that octadecyl tyrosine hydrochloride (OTH) functions as an adjuvant when complexed with tetanus toxoid and formalin inadtivated type I, II and III poliomyelitis virus vaccine. Nixon-George et al. ((1990) *J*. *Immunol*. 14:4798) have also reported that octadecyl esters of aromatib amino acids complexed with a recombinant hepatitis B surface antigen enhanced the host immune responses against hepatitis B virus.

Adjuvant compounds may also be chosen from the polymers of acrylic or methacrylic acid and the copolymers of maleic anhydride and alkenyl derivative. Adjuvant compounds are the polymers of acrylic or methacrylic acid which are cross-linked, especially with polyalkenyl ethers of sugars or polyalcohols. These compounds are known by the term carbomer (Pharmeuropa Vol. 8, No. 2, June 1996). Preferably, a solution of adjuvant according to the invention, especially of carbomer, is prepared in distilled water, preferably in the presence of sodium chloride, the solution obtained being at acidic pH. This stock solution is diluted by adding it to the desired quantity (for obtaining the desired final concentration), or a substantial part thereof, of water charged with NaCI, preferably physiological saline (NaCl 9g/l) all at once in several portions with concomitant or subsequent neutralization (pH 7.3 to 7.4), preferably with NaOH. This solution at physiological pH will be used as it is for mixing with the immunizing agent; said mixture being amenable to storage in the freeze-dried, liquid or frozen form.

Persons skilled in the art can also refer to U.S. Patent No. 2,909,462 (incorporated herein by reference) which describes adjuvants encompassing acrylic polymers cross-linked with a polyhydroxylated compound having at least 3 hydroxyl groups (preferably not more than 8), the hydrogen atoms of the at least three hydroxyls being replaced by unsaturated aliphatic radicals having at least 2 carbon atoms. The preferred radicals are those containing from 2 to 4 carbon atoms (e.g. vinyls, allyls and other ethylenically unsaturated groups). The unsaturated radicals may themselves contain other substituents, such as methyl. The products sold under the name Carbopol (BF Goodrich, Ohio, USA) are particularly appropriate. They are cross-linked with allyl sucrose or with allyl pentaerythritol. Among them, there may be mentioned Carbopol (for example, 974P, 934P and 971 P). Among the copolymers of maleic anhydride and alkenyl derivative, the copolymers EMA (Monsanto; which are copolymers of maleic anhydride and ethylene, linear or cross-linked, (for example cross-linked with divinyl ether)) are preferred. Reference may be made to J. Fields et al. ((1960) *Nature* 186:778) for a further description of these chemicals (incorporated (herein by reference).

In further aspects of this invention, adjuvants useful for parenteral administration of immunizing agent include aluminum compounds (such as aluminum hydroxide, aluminum phosphate, and aluminum hydroxy phosphate; but might also be a salt of calcium, iron or zinc, or may be an insoluble suspension of acylated tyrosine, or acylated sugars, cationically or anionically derivatised polysaccharides, or polyphosphazenes). The antigen can be precipitated with, or adsorbed onto, the aluminum compound according to standard protocols well known to those skilled in the art.

Other adjuvants encompassed by embodiments of this invention include lipid A (in particular 3-de-0-acylated monophosphoryl lipid A (3D-MPL). 3D-MPL is a well known adjuvant manufactured by Ribi lmmunochem, Montana. Chemically it is often supplied as a mixture of 3-de-0-acylated monophosphoryl lipid A with 4, 5, or 6 acylated chains. It can be prepared by the methods taught in GB 21222048. A preferred form of 3D-MPL is in the form of a particulate formulation having a particle size less than 0.2pm in diameter (European Patent EP 689454).

Adjuvants for mucosal immunization may include bacterial toxins (e.g., the cholera toxin (CT), the *E. coli* heat-labile toxin (LT), the *Clostrilium difficile* toxin A and the pertussis toxin (PT), or combinations, subunits, toxoids, or mutants thereof). For example, a purified preparétion of native cholera toxin subunit B (CTB) can be of use. Fragments, homologs, derivatives, and fusion to any of these toxins are also suitable, provided that they retain adjuvant activity. A mutant having reduced toxicity may be used. Mutants have been described (e.g., in WO 95/17211 (Arg-7-Lys CT mutant), WO 96/6627 (Arg 192-Gly LT mutant), and WO 95134323 (Arg-9-Lys and Glu-129-Gly PT mutant)). Additional LT mutants include, for example Ser-63-Lys, Ala-69-Gly, Glu-1 10-Asp, and Glu-1 12-Asp mutants. Other adjuvants (such as a bacterial monophosphoryl lipid A (MPLA)) of various sources (e.g., *E. coli, Salmone*/*Ia minnesota, Salmonella typhimurium,* or *Shigella flexneri)* can also be used in the mucosal administration of immunizing agents.

Adjuvants useful for both mucosal and parenteral immunization include polyphosphazene (for example, WO 95/2415), DC-chol (3 b-(N-(N',N'-dimethyl aminomethane)-carbamoyl) cholesterol (for example, U.S. Patent No. 5,283,185 and WO 96/14831) and QS-21 (for example, WO 88/9336).

Adjuvants/immunostimulants as described herein may be formulated together with carriers, such as for example liposomes, oil in water emulsions, and/or metallic salts including aluminum salts (such as aluminum hydroxide). For example, 3D-MPL may be formulated with aluminum hydroxide (as discussed in EP 689454) or oil in water emulsions (as discussed in WO 9517210); QS2I may be advantageously formulated with cholesterol containing liposomes (as discussed in WO 9633739), in oil water emulsions (as discussed in WO 9517210) or alum (as discussed in WO 9815287). When formulated into vaccines, immunostimulatory oligonucleotides (i.e. CpGs) are generally administered in free solution together with free antigen (as discussed in WO 9602555; McCluskie and Davis (1998) *Supra),* covalently conjugated to an antigen (as discussed in WO 9816247), or formulated with a carrier such as aluminum hydroxide or alum (as discussed in Davies et al. *Supra;* Brazolot-Millan et al (1998) *Proc. Natl. Acad. Sci.* 95:15553).

Combinations of adjuvants/immunostimulants are also within the scope of this invention. For example, a combination of a monophosphoryl lipid A and a saponin derivative (as described in WO 9400153, WO 9517210, WO 9633739, WO 9856414, WO 9912565, WO 9911214) can be used, or more particularly the combination of QS21 and 3D-MPL (as described in WO 9400153). A combination of an immunostimulatory oligonUcleotide and a saponin (such as QS2I), or a combination of monophosphoryl lipid A (preferably 3D-MPL) in combination with an aluminum salt also form a potent adjuvant for use in the present invention.

The following non-limiting examples are illustrative of the present invention:

### EXAMPLES

### Example 1: Generation of a Modified CEA Gene

T-cell epitopes within the native CEA protein include CAP-1; said epitope comprising the amino acid sequence YLSGANLNL (Tsang et al. (1995) *J*. *Natl. Cancer Inst* 87:982-990). As previously noted, the immunogenicity of this epitope (in the form of a peptide) was increased by changing position six of the CAP-1 epitope from N (asparagine) to D (aspartic acid) (Zaremba et al. (1997) *Cancer Res.* 57:4570-4577). Using standard techniques of *in vitro* mutagenesis (Ausubel et al. (1997) *Curr. Protocols in Mol. Blot),* this modification was introduced into the full-length CEA gene via mutation of the codon AAC (which encodes asparagine) to GAC (which encodes aspartic acid). The resulting modified CEA gene now comprises the modified epitope YLSGADLNL (the change from N to D is shown in bold). This modified CEA gene, encoding for a protein with aspartic acid (D) in place of asparagine (N) in the sixth amino acid position of the CAP-1 epitope, is designated CEA (6D) (for example, see SEQ ID NO: 1 (Figure 1)).

### Example 2: Generation of Recombinant Fowlpox. Vaccinia and MVA Constructs

The generation of recombinant poxviruses is accomplished via homologous recombination between poxvirus genomic DNA and a plasmid vector that carries the heterologous sequences to be inserted. Plasmid vectors for the insertion of foreign sequences into poxviruses are constructed by standard methods of recombinant DNA technology (for example, Ausubel et al. (1997) *Curr. Protocols. Mol. Biol).* The plasmid vectors contain one or more chimeric genes, each comprising a poxvirus promoter linked to a protein coding sequence, flanked by viral sequences from a non-essential region of the poxvirus genome. The plasmid is transfected into cells infected with the parental poxvirus, and recombination between poxvirus sequences on the plasmid and the corresponding DNA in the viral genome results in the insertion into the viral genome of the chimeric genes on the plasmid. Recombinant viruses are selected and purified using any of a variety of selection or screening systems (Mazzara et al. (1993) *Meth. Enzymol*. 217:557-581; Jenkins et al. (1991) *AIDS Res. Hum. Retroviruses* 7:991-998; Sutter et al. (1994) *Vaccine* 12:1032-1040). Insertion of the foreign genes into the vaccinia genome is typically confirmed by polymerase chain reaction (PCR) analysis. Expression of the foreign genes is demonstrated by Western analysis and/or via immunoprecipitation of expressed products.

### Generation of Recombinant Poxviruses

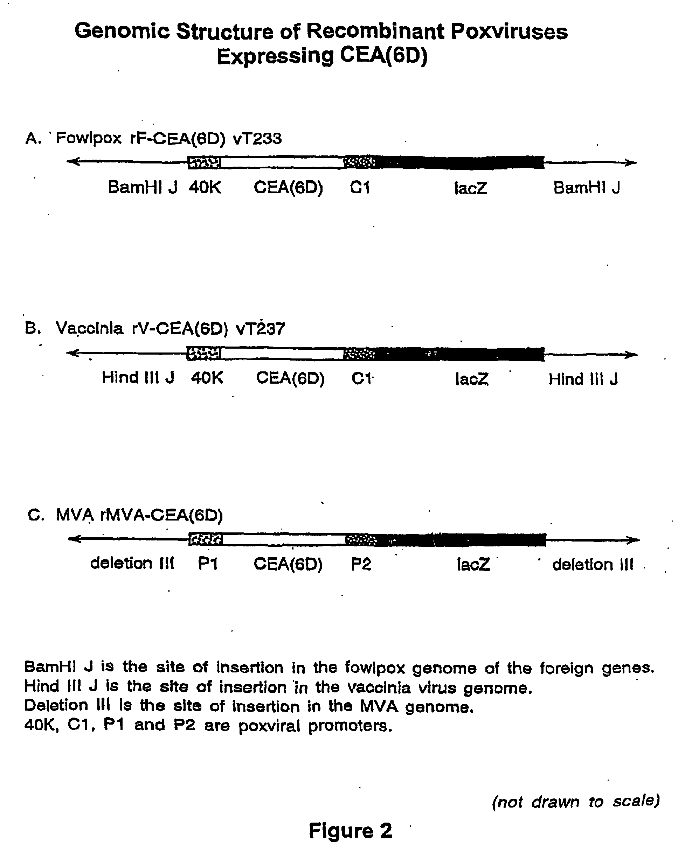

For the generation of recombinant fowlpox virus rF-CEA(6D), a plasmid vector designated pT5071 was constructed to direct insertion of the foreign sequences into the BamHl J region of the fowlpox genome. The CEA(6D) gene is under the control of the vaccinia 40K promoter (Gritz et al. (1990) *J. Virol*. 64:5948-5957). In addition, the *E. coil l*acZ gene, under the control of the fowlpox virus C1 promoter (Jenkins et al. (1991) *AIDS* Res. *Hum. Retroviruses* 7:991-998), is included as a screen for recombinant progeny. These foreign sequences are flanked by DNA sequences from the BamHI J region of the fowlpox genome. A plaque-purified isolate from the POXVAC-TC strain of fowlpox was used as the parental virus for this recombinant vaccine. The geheration of recombinant fowlpox virus was accomplished via homologous recombinant between fowlpox sequences in the fowlpox genome and the corresponding sequences in pT5071 in fowlpox-infected primary chick embryo dermal cells transfected with pT5O71. Recombinant virus was identified using a chromogenic assay preformed on viral plaques *in situ*. This assay detects expression of the lacZ gene product in the presence of halogenated indolyl-beta-D-galactoside (Bluo-gal) (Chakrabarti et al. (1985) *Mol*. *Cell Bio* 3403-3409). Viral plaques expressing *IacZ* appear blue against a clear background. Positive plaques, designated vT233, were picked from the cell monolayer and their progeny replated. Seven rounds of plaque isolation and replating in the presence of Bluo-Gal resulted in the purification of the desired recombinant. A schematic representation of the genomic structure of vT233 is depicted in Figure 2(A).

For the generation of recombinant vaccinia rV-CEA(6D), a plasmid vector designated pT2 146 was constructed to direct insertion of the foreign sequences into the thymidine kinase gene (located in the Hind Ill J region of the vaccinia genome). The CEA(6D) gene is under the transcriptional control of the vaccinia 40K promoter and the *E. coIl. IacZ* gene is under the control of the fowlpox virus Ci promoter. These foreign sequences are flanked by DNA sequences from the Hind Ill J region of the vaccinia genome. A plaque-purified isolate from the Wyeth (New York City Board of Health) strain of vaccinia was used as the parental virus for this recombinant vaccine. The generation of recombinant vaccinia virus was accomplished via homologous recombination between vaccinia sequences in the Wyeth vaccinia genome and the corresponding sequences in pT2146 in vaccinia-infected cells transfected with pT2146. Recombinant virus was identified using the chromogenic assay for the *lacZ* gene product (previously described). Positive plaques, designated vT237, were picked from the cell monolayer and their progeny replated. Five rounds of plaque isolation and replating in the presence of Bluo-Gal resulted in the purification of the desired recombinant. A schematic representation of the genomic structure of vT237 is depicted in Figure 2(6).

For the generation of a recombinant MVA that expressed CEA(6D), a plasmid vector was constructed to direct insertion of the foreign sequences into the MVA genome. The CEA(6D) gene and the *E. coil* lacZ gene were each under the control of a poxviral promoter. These foreign sequences were flanked by DNA sequences from the MVA genome into which the foreign sequences were to be inserted (for example, deletion Ill (Sutter et at (1994) Vaccine 12:1032-1040)). The generation of recombinant MVA was accomplished via homologous recombination between MVA sequences in the MVA gehome and the corresponding sequences in the plasmid vector in MVA-infected cells transfected withthe plasmid vector. Recombinant plaques were picked from the cell monolayer under selective conditions and their progeny further propagated. Additional rounds of plaque isolation and replating resulted in the purification of the desired recombinant virus. A schematic representation of the genomic structure of the MVA recombinant construct is depicted in Figure 2(C).

### Example 3: Generation of anAI_VAC(2)-modified CEA-B7.1 (Human) Recombinant Construct frCP1586)

To generate ALVAC(2)-CEAmod/hB7.1, a coding sequence containing the H6 (Perkins et al. (1989) *J*. *Virol*. 63:3829-3936) promoted modified CEA and the human 67.1 gene (under the control of synthetic Early/Late promoter) was subcloned into a generic ALVAC donor plasmid specific for the C5 insertion site (see below). The donor plasmici was then used to insert the expression cassette into the CS insertion locus in the ALVAC(2) genome by *in vitro* recombination (Piccini et al. (1987) *Meth. Enzymol*. 153:545).

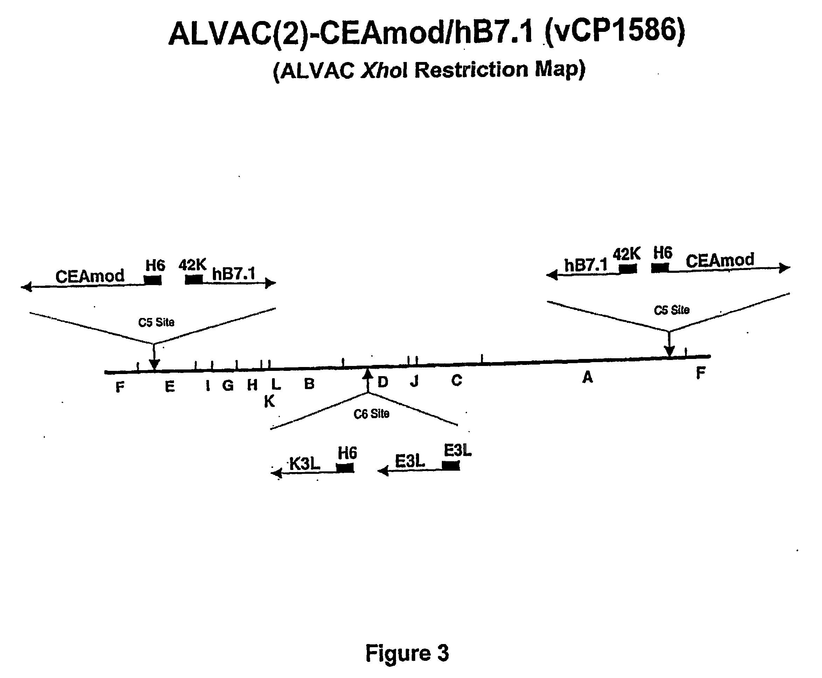

The locus designated CS was used for the insertion of the modified CEA and human B7.1 coding sequences into the ALVAC(2) vector. By virtue of the C5 locus existing within the extensive inverted terminal repetitions (ITRs) of the virus genome, insertion into this locus results in the occurrence of two copies of the inserted sequence. A schematic of the redombinant construct is depicted in Figure 3. [Presently, no function has been ascribed to the CS encoded polypeptide nor does the deduced amino acid open reading frame encoded in this region share significant homology to any entry in the existing protein databases.]

### Particulars of the Generation of the vCPIS86 ALVAC recombinant

### (I) Modified CEA:

sequence (up to the Bg/II site) by PCR amplification from plasmid pT2147 (comprising a full length sequence of the modified CEA(6D)) using the following PCR primers:

HM102: 5	'-TTG-TCC-GAG-CTC-TCG-CGA-TAT-CCG-TTA-AGT-TTG-
TAT CGT-AAT-GGA-GTC-TCC-CTC-GGC-CCC-3'  (Sequence ID
No. 3)

HM103: 5'-CCG-GAA-TTC-TCA-CAA-GAT-CTG-ACT-TTA-TGA-C-3'
(Sequence ID NO. 4)

The PCR product was digested with *Sac*I and *Eco*R1 to generate compatible ends for cloning into *Sac*I, *Eco*R1 and Shrimp Alkaline Phosphatase digested pBSK+ vector (DH5α cells). The resulting plasmid (designated pF102.103) was sequenced to confirm the fidelity of the insert. The remainder of the CEA(6D) sequence was isolated by digestion of plasmid pT2147 with *Bg*/II and Sa/I. The resulting 1.7Kbp fragment contained the 3'end of CEA(6D). This fragment was ligated to *Bg*/II, *Sal*I and Shrimp Alkaline Phosphatase digested pF102.103 plasmid and transformed into DH5α cells. The resulting plasmid was designated p3'H6MCEA.

An ALVAC donor plasmid was constructed by transferring the 3'H6-modified CEA sequence fragment from p3'H6MCEA to ALVAC insertion plasmid NVQH6MC5. NVQH6MC5 was initially made via digestion of CS donor plasmid NVQH6C5LSP-18 within its polylinker region with *BamHl*. It was subsequently treated with alkaline phosphatase and ligated to kinased and annealed oligonucleotides SPCSPL1
(5'-GAT-CGT-CGA-CGA-GCT-CGA-ATT-CG-3') (Sequence ID No. 5) and SPC5PL2 (S'-GAT-CCG-AATTCG-ACC-TCG-TCG-AC-3') (Sequence ID No. 6) - thus generating plasmid NVQH6MC5.

Plasmid p3'H6MCEA was digested with *Nru*I and *Sal*I*;* the fragment containing the 3'H6 modified CEA sequence purified and subsequently ligated to *Nru*I*, Xho*I and Shrimp Alkaline Phosphatase digested NVQH6MC5 vector. The resulting donor plasmid, containing the modified CEA coding sequences under the control of a full length H6 promoter, was designated pAH6MCEA.

### (ii) B7.1

The following primers were used to fuse the 42K promoter and PCR out the entire B7.1 gene from plasmid pT2147:

H Ml 04: 5'-TTG-TCC-GAG-CTC-GAA-TTC-TTT-ATT-G GG-AAG-AAT-ATG-
ATA-ATA-TTT-TGG-GAT-TTC-AAA-ATT-GAA-AAT-ATA-TAA-TTACAA-
TAT-AAA-ATG-GGC-CAC-ACA-CGG-AGG-CAG-3' (Sequence ID No. 7).
HMI05: 5'-ACG-GCA-GTC-GAC-TTA-TAC-AGG-GCG-TAC-ACT-3' (Sequence
ID No. 8).

Primer HM1O4 contained the entire sequence of the 42K promoter. The resulting PCR product (designated F104.105) was digested with *Eco*RI and *Sa*/I, and subsequently ligated into *Eco*RI, *Sa*II and Shrimp Alkaline Phosphatase digested pBSK+ vector(SURE cells), The resulting plasmid was designated pF104.105 and was sequenced to confirm the fidelity of the insert.

### (iii) Donor Plasmid pA2147

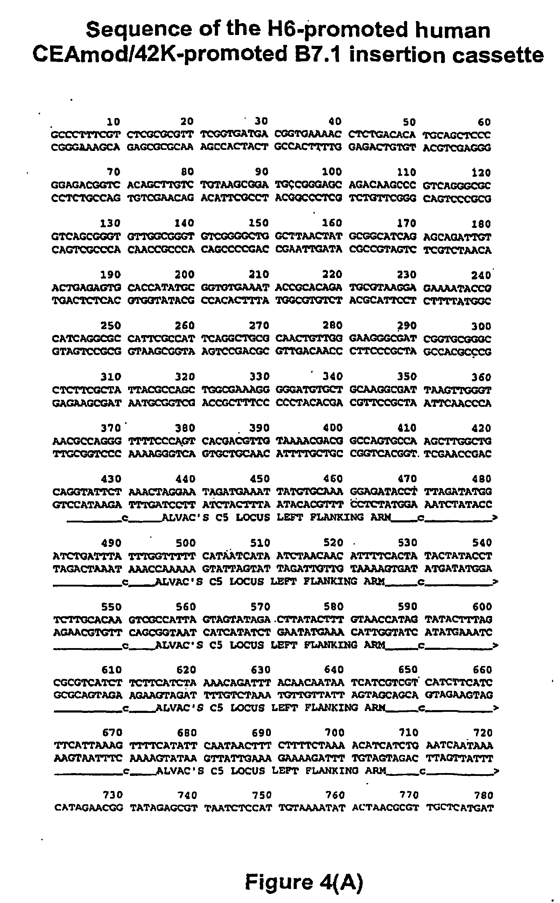

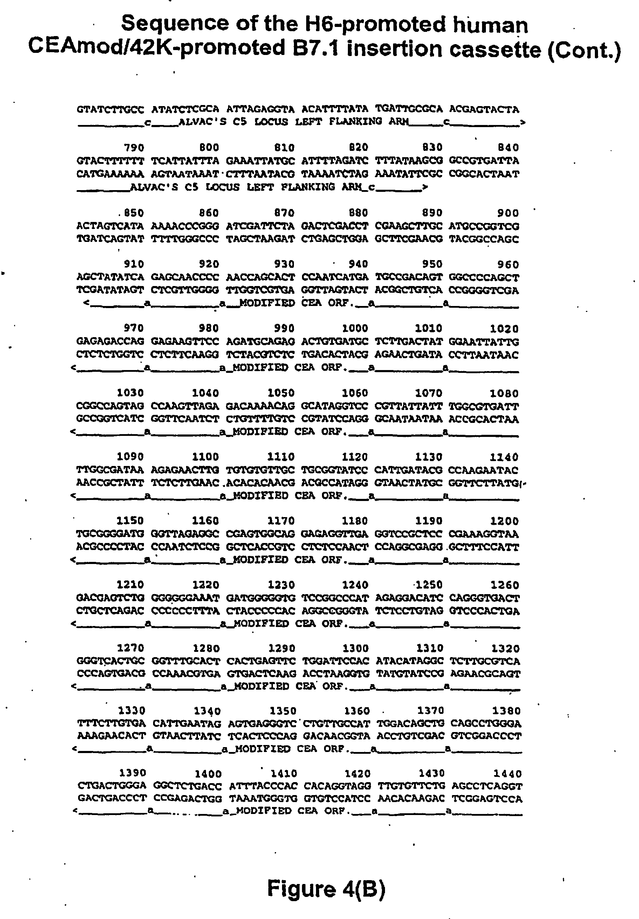

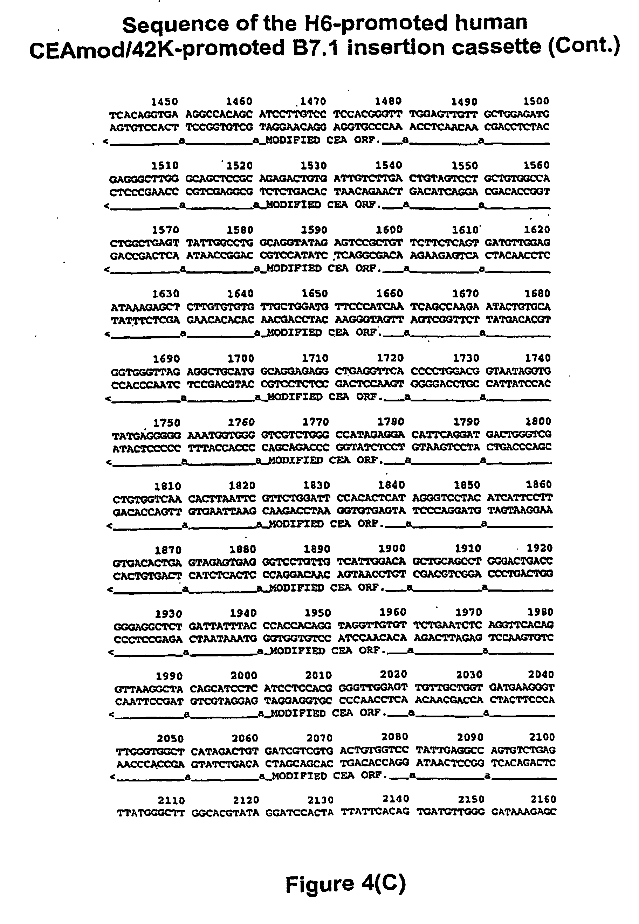

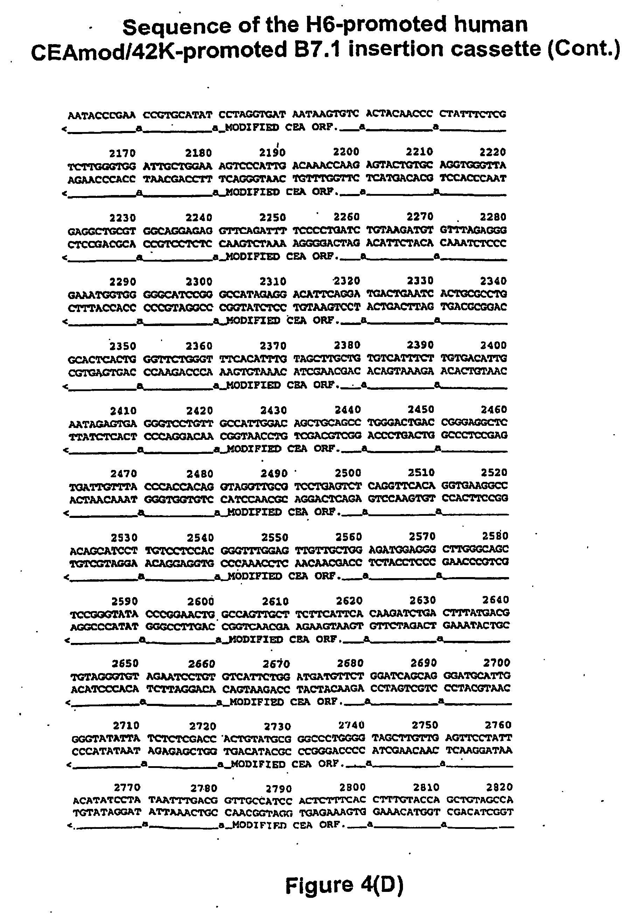

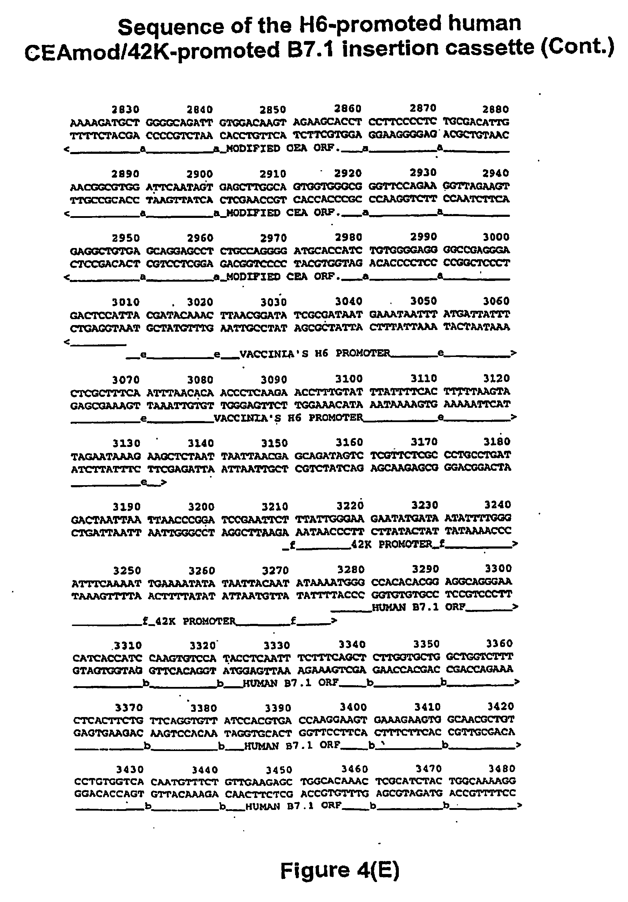

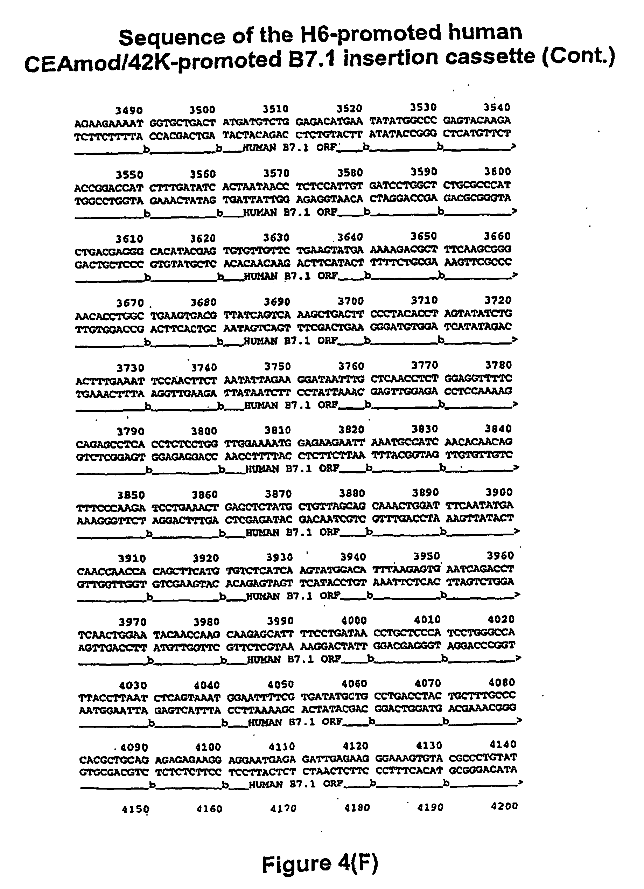

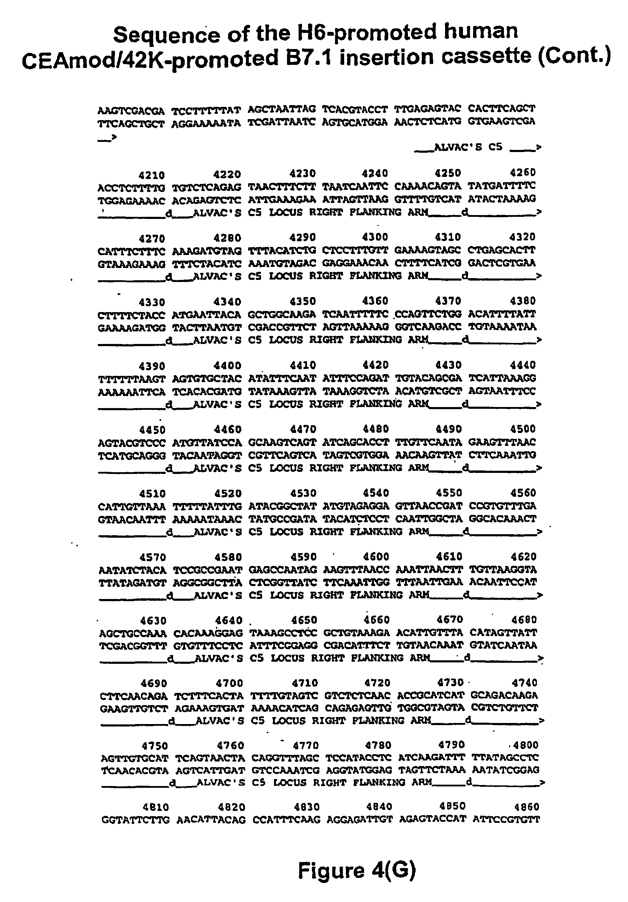

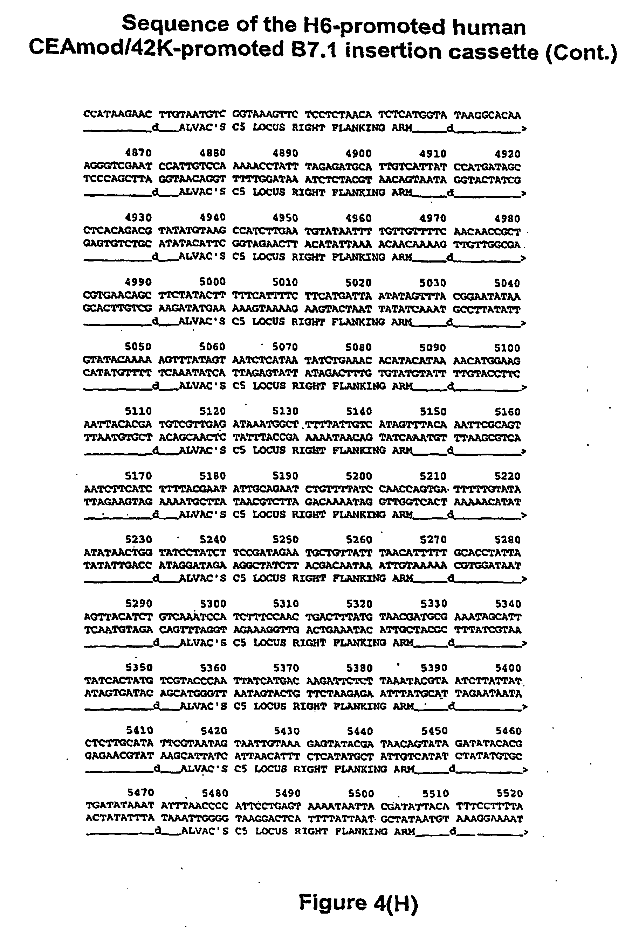

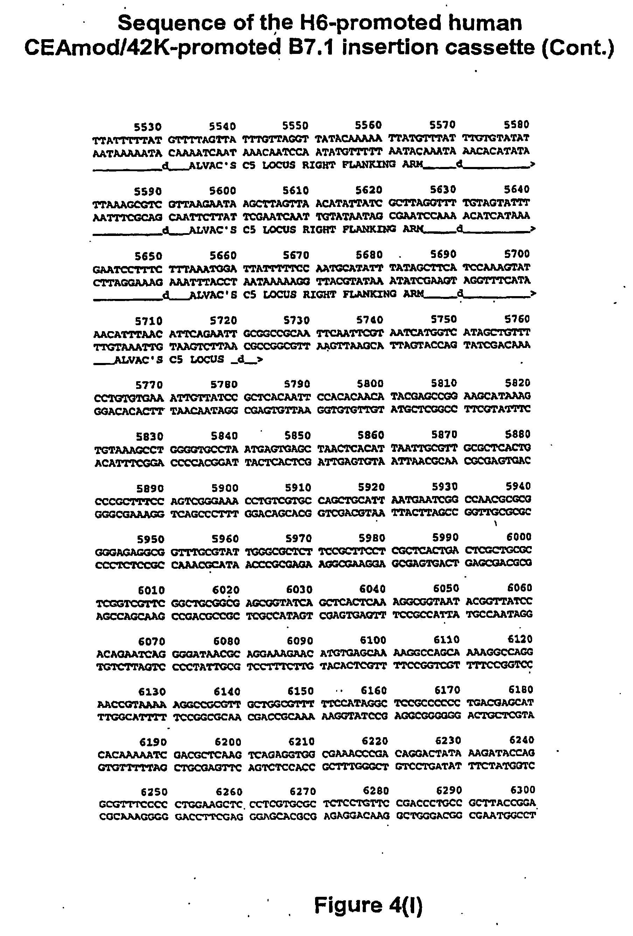

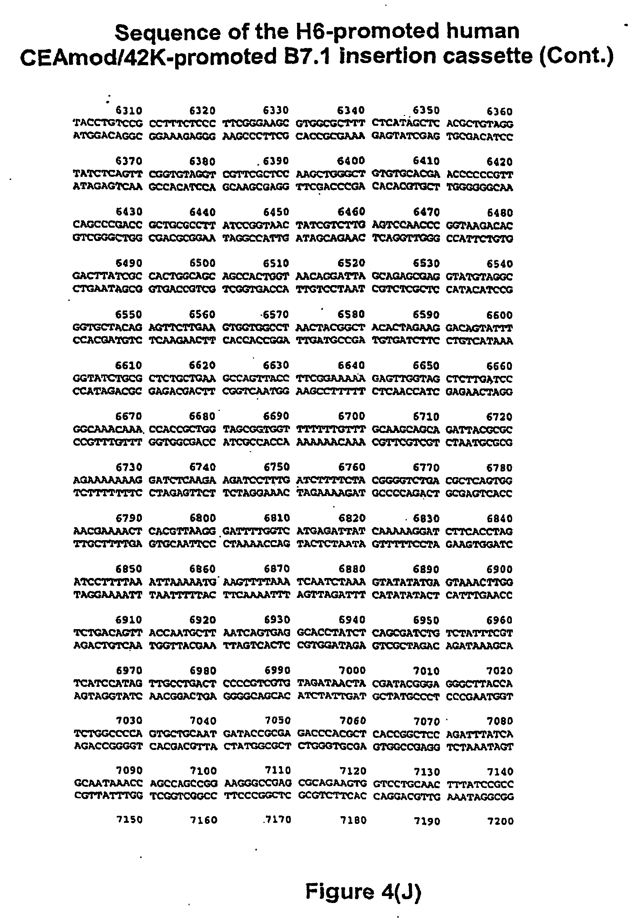

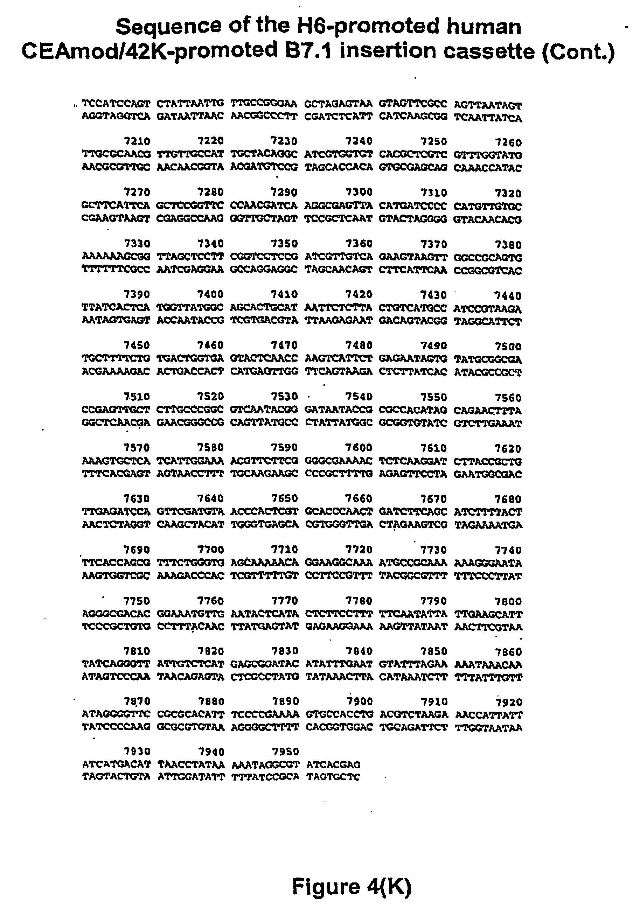

The fragment containing the 42K-67.1 coding sequences was excised from pF104.105 by digestion with *Eco*RI and *Sa*/I, and subsequently purified. This fragment was ligated to *Eco*RI, *Sa*/I, Shrimp Alkaline Phosphatase digested pAH6MCEA plasmid and transformed into SURE cells. The resulting donor plasmid was designated pA2147. (See Figure 4 for the sequence of the H6-promoted human modified CEA/42K promoted B7.1 cassette of pA2147 for insertion into ALVAC.)

### (iv) Generation of VCP1586

Recombination between donor plasmid pA2147 and ALVAC(2) rescuing virus generated recombinant virus vCP1586. This recombinant viral construct comprised the vaccinia H6 promoted modified human CEA gene sequence and the 42K promoted human 67.1 gene sequence in the C5 locus (for ALVAC insertion and inserted DNA sequence particulars, see Figures 3 and 4). Recombination was performed utilizing procedures described in the art and known to skilled artisans (Piccini et al. (1987), *supra;* other procedures can also be utilized as described above and/or as described, for example, in U.S. Patent Nos. 4769330, 4722848, 4603112, 5174993, 5110587, all of which are incorporated herein by reference).

### Verification of insertion

### (I) Restriction Enzyme Analysis

Viral genomic DNA was isolated from cells infected with vCP1586 pursuant to methods well known to those skilled in the art (for example, as taught in *Current Protocols in Molecular Biology,* F.M. Ausubel et al. (Eds.), John Wiley and Sons, Inc., N.Y., U.S.A. (1998); *Molecular Cloning: A Laboratory Manual (2*^{*nd*} *Ed.),* J. Sambrook, E.F. Fritsch and T. Maniatis (Eds.), Cold Spring Harbor Laboratory Press, N.Y., U.S.A. (1989)). The viral genomic DNA was digested with restriction endonucleases *(HindIII,* Pst I, BamH1 or *Xhol).* The resultant DNA fragments were fractionated by electrophoresis through an agarose gel and visualized by ethidium bromide staining. The insertion of the modified CEA and B7.1 expression cassette at the CS locus was confirmed (for example, see Figure 3 for a schematic representation of the *Xhol* restriction map of vCPI 586).

### (ii) Immunoprecipitation

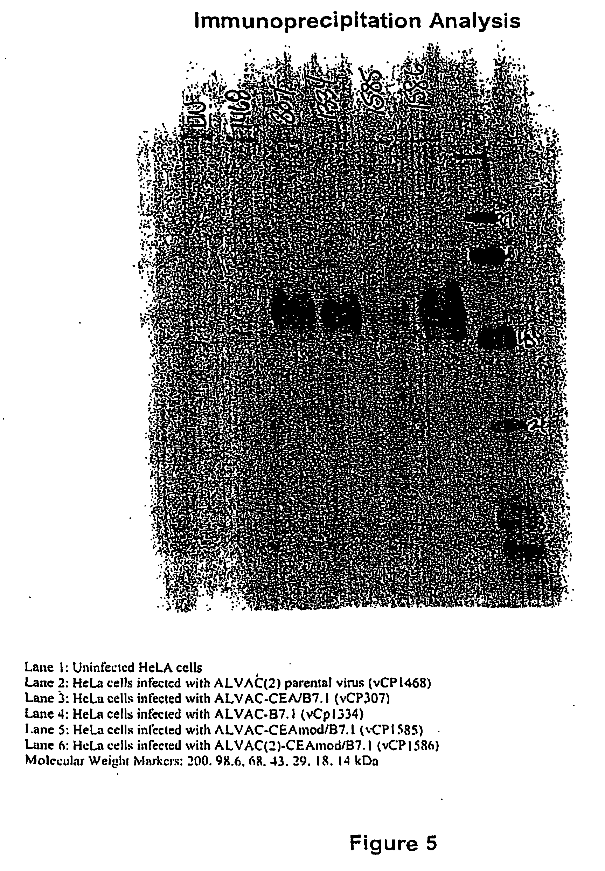

Immunoprecipitation analyses were performed using radiolabeled lysates derived from uninfected HeLa cells or cells infected with either ALVAC(2) parental virus (vCP1486), ALVAC-CEA/B7 (vCP3O7), ALVAC-hB7.1 (vCP11334), ALVAC-CEAmod/hB7.1 (vCP1585) or ALVAC(2)-CEAmod/hB7.1 (vCP1586) as previously described (Taylor et al. (1990) *J. Virol.* 64:1441-1450). Briefly, HeLa cell cultures were infected at a multiplicity of infection (m.o.i.) of 10 pfu/cell in methionine and cystine-free media supplemented with [³⁵S]-methionine/cysteine (30µCi/mi). Cells were lysed at 18 hours post-infection. Immunoprecipitation was performed using a B7.1 specific monoclonal antibody (HB71; ATCC#80991). immunoprecipitates were fractionated on a 10% SDS-Polyacrylamide gel. The gel was fixed and treated for fluorography with 1 M Na-salicylate for 1/2 hr. The dried gel was exposed to Kodak XAR-2 film to visualize the protein species. Results with anti-hB7.1 demonstrate expression of human B7 in HeLa cells infected with ALVAC(2)-CEAmod/hB7.1 (vCP1586) and the other ALVAC-based recombinants expressing hB7.1, but not in parentally infected cells (Figure 5).

### (iii) Western Blot Analysis

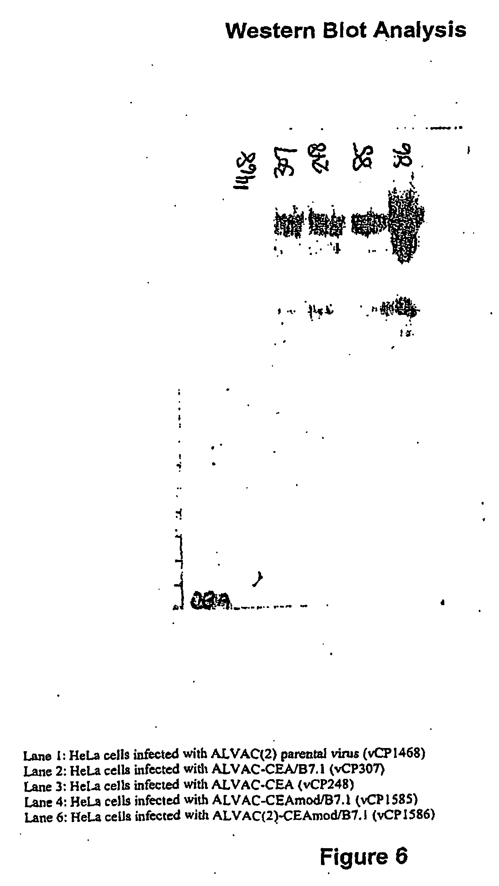

HeLa cells were infected for 18 hours at a multiplicity of 10 pfu/cell with either ALVAC(2) parental virus (vCP1486), ALVAC-CEA/B7 (vCP3O7), ALVAC-CEA (vCP248), ALVAC-CEAmod/hB7.1 (vCP 1585) or ALVAC(2)-CEAmod/hB7. 1 (vCPI 586). Cell lysates were separated by SDS-PAGE and transferred to nitrocellulose utilizing standard techniques. The blot was incubated with anti-CEA monoclonal antibody Col-1 (1/1 000 dilution), followed by HRP conjugated rabbit anti-mouse utilizing HRP peroxide substrate with DAB/Metal (Pierce). The results obtained demonstrate the expression of full length CEA in HeLa cells infected with ALVAC(2)-CEAmod/hB7.1 (vCP1586) and other ALVAC-based recombinants expressing CEA (Figure 6).

Having illustrated and described the principles of the invention in particular and/or preferred embodiments, it should be appreciated by those skilled in the art that the invention can be modified in arrangement and detail without departure from such principles. We claim all modifications coming within the scope of the following claims. All publications, patents and patent applications referred to herein, are herein incorporated by reference in their entirety to the same extent as if each individual publication, patent or patent application was specifically and individually indicated to be incorporated by reference in its entirety.

## Claims

1. A CEA agonist polypeptide comprising a modified epitope of CEA, wherein said modified epitope contains the sequence YLSGADLNL.

2. The CEA agonist polypeptide of claim 1 having the sequence of SEQ ID NO: 1 (Figure 1).

3. A nucleic acid comprising a nucleic acid sequence which encodes the CEA agonist polypeptide of any one of claims 1 or 2.

4. The nucleic acid of claim 3 comprising the sequence of SEQ ID NO: 2 (Figure 1).

5. The nucleic acid of claim 3 having the sequence of SEQ ID NO: 2 (Figure I).

6. The nucleic acid of any one of claims 3 through 5 wherein the nucleic acid is selected from viral nucleic acid, bacterial DNA, plasmid DNA, naked/free DNA, and RNA.

7. The nucleic acid of claim 6 wherein the viral nucleic acid is selected from the group consisting of adenoviral, alphaviral, and poxviral nucleic acid.

8. The poxviral nucleic acid of claim 7, wherein said nucleic acid is selected from the group consisting of avipox, orthopox, and suipox nucleic acid.

9. The poxviral nucleic acid of claim 7, wherein said nucleic acid is selected from the group consisting of vaccinia, fowlpox, canary pox and swinepox.

10. The poxviral nucleic acid of claim 7, wherein said nucleic acid is selected from the group consisting of TROVAC, NYVAC, ALVAC, MVA, Wyeth, and Poxvac-TC.

11. The nucleic acid of any one of claims 3 through 10 further comprising a nucleic acid sequence encoding at least one member selected from the group comprising cytokines, lymphokines, and co-stimulatory molecules.

12. A vector comprising the nucleic acid of any one of claims 3 through 11.

13. The vector of claim 12, wherein said vector is selected from the group consisting of recombinant viruses and bacteria.

14. The recombinant virus vector of claim 13, selected from the group consisting of adenovirus, alphavirus and poxvirus.

15. The poxvirus of claim 14, selected from the group consisting of avipox, orthopox, and suipox.

16. The poxvirus of claim 14, selected from the group consisting of vaccinia, fowlpox, canary pox, and swinepox.

17. The poxvirus of claim 14, selected from the group consisting of TROVAC, NYVAC, ALVAC, MVA, Wyeth, and Poxvac-TC.

18. The vector of any one of claims 12 through 17 further comprising a nucleic acid sequence encoding at least one member selected from he group consisting of cytokines, lymphokines, and co-stimulatory molecules.

19. A cell comprising a nucleic acid according to any one of claims 3 through 11 wherein the cell expresses a CEA agonist polypeptide.

20. The cell of claim 19 wherein the cell additionally expresses a MHC HLA class I molecule.

21. The cell of claim 19 wherein the cell is an antigen-presenting cell.

22. The cell of claim 21 wherein the cell is a dendritic cell.

23. A mixture comprising a nucleic acid according to any one of claims 3 through 11 and at least one member selected from the group consisting of cytokines, lymphokines, immunostimulatory molecules, and nucleic acids coding therefor.

24. A mixture comprising a vector according to any one of claims 12 through 18 and at least one member selected from the group consisting of cytokines, lymphokines, immunostimulatory molecules, and nucleic acids coding therefor.

25. A mixture comprising a CEA agonist polypeptide according to any one of claims 1 or 2 and at least one member selected from the group consisting of cytokines, lymphokines, immunostimulatory molecules, and nucleic acids coding therefor.

26. A mixture comprising a cell according to any one of claims 19 through 22 and at least one member selected from the group consisting of cytokines, lymphokines, immunostimulatory molecules, and nucleic acids coding therefor.

27. A pharmaceutical composition comprising a CEA agonist polypeptide according to any one of claims 1 or 2, and a pharmaceutically acceptable diluent or carrier.

28. A pharmaceutical composition comprising a nucleic acid according to any one of claims 3 through 11, and a pharmaceutically acceptable diluent or carrier.

29. A pharmaceutical composition comprising a vector according to any one of claims 12 through 18, and a pharmaceutically acceptable diluent or carrier.

30. A pharmaceutical composition comprising a cell according to any one of claims 19 through 22, and a pharmaceutically acceptable diluent or carrier.

31. A pharmaceutical composition comprising a mixture according to any one of claims 23 through 26, and a pharmaceutically acceptable diluent or carrier.

32. The pharmaceutical composition of any one of claims 27 through 31 further comprising an adjuvant.

33. A method of inducing an immune response in an animal directed against a member selected from the group consisting of:
a CEA protein or fragment thereof,
a CEA agonist polypeptide according to any one of claims 1 or 2,
a CEA epitope,
a modified CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a CEA agonist polypeptide or fragment thereof according to any one of claims 1 or 2 in an amount sufficient to induce an immune response.

34. A method of inducing an immune response in an animal directed against a member selected from the group consisting of:
a CEA protein or fragment thereof,
a CEA agonist polypeptide according to any one of claims 1 or 2,
a CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a nucleic acid according to any one of claims 3 through 11 in an amount sufficient to induce an immune response.

35. A method of inducing an immune response in an animal directed against a member selected from the group consisting of:
a CEA protein or fragment thereof,
a CEA agonist polypeptide according to any one of claims 1 or 2,
a CEA epitope,
a modified CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a vector according to any one of claims 12 through 18 in an amount sufficient to induce an immune response.

36. A method of inducing an immune response in an animal directed against a member selected from the group consisting of:
a CEA protein or fragment thereof,
a CEA agonist polypeptide according to any one of claims 1 or 2,
a CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a cell according to any one of claims 19 through 22 in an amount sufficient to induce an immune response.

37. A method of inducing an immune response in an animal directed against a member selected from the group consisting of:
a CEA protein or fragment thereof,
a CEA agonist polypeptide according to any one of claims 1 or 2,
a CEA epitope,
a modified CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a mixture according to any one of claims 23 through 26 in an amount sufficient to induce an immune response.

38. A method of inducing an immune response in an animal directed against a member selected from the group consisting of:
a CEA protein or fragment thereof,
a CEA agonist polypeptide according to any one of claims 1 or 2,
a CEA epitope,
cells expressing a CEA protein or fragment thereof, CEA agonist polypeptide according to any one of claims 1 or 2, CEA epitope, modified CEA epitope, and
cells binding a CEA protein or fragment thereof, CEA agonist polypeptide according to any one of claims 1 or 2, CEA epitope, modified CEA epitope
comprising administering to said animal a composition according to any one of claims 27 through 32 in an amount sufficient to induce an immune response..

39. A method of inhibiting a CEA epitope expressing carcinoma cell in a patient comprising administering to said patient an effective amount of the CEA agonist polypeptide according to any one of claims 1 or 2.

40. A method of inhibiting a CEA epitope expressing carcinoma cell in a patient comprising administering to said patient an effective amount of the nucleic acid according to any one of claims 3 through 11.

41. A method of inhibiting a CEA epitope expressing carcinoma cell in a patient comprising administering to said patient an, effective amount of the vector according to any one of claims 12 through 18.

42. A method of inhibiting a CEA epitope expressing carcinoma cell in a patient comprising administering to said patient an effective amount of the cell according to any one of claims 19 through 22.

43. A method of inhibiting a CEA epitope expressing carcinoma cell in a patient comprising administering to said patient an effective amount of the mixture according to any one of claims 23 through 26.

44. A method of inhibiting a CEA epitope expressing carcinoma cell in a patient comprising administering to said patient an effective amount of the composition according to any one of claims 27 through 32.

45. The methods of any one of claims 39 through 44 wherein the carcinoma cell is a gastrointestinal, breast, pancreatic, bladder, ovarian, lung or prostate carcinoma cell.

46. A treatment for cancer comprising any one of the methods of claims 39 to 45.

47. The use of a CEA agonist polypeptide of claim 1 for the manufacture of a medicament for the treatment of cancer.

48. The use of claim 47, wherein said polypeptide comprises the amino acid sequence of SEQ. ID NO. 1.

49. The use of an isolated, purified or recombinant nucleic acid sequence having the sequence of SEQ. ID NO. 2 for the manufacture of a medicament for the treatment of cancer.
